(19) Europäisches Patentamt
European Patent Office
Office européen des brevets

(11) **EP 4 162 923 A1**

(12) **DEMANDE DE BREVET EUROPEEN**

(43) Date de publication:
**12.04.2023 Bulletin 2023/15**

(21) Numéro de dépôt: **22210622.1**

(22) Date de dépôt: **10.11.2021**

(51) Classification Internationale des Brevets (IPC):
**A61K 8/37** (2006.01)     **A61K 8/35** (2006.01)
**A61K 8/41** (2006.01)     **A61K 8/49** (2006.01)
**A61Q 17/04** (2006.01)

(52) Classification Coopérative des Brevets (CPC):
**A61K 8/4966; A61K 8/35; A61K 8/37; A61K 8/415;
A61K 8/496; A61Q 17/04;** A61K 2800/30

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **12.11.2020 FR 2011622**

(62) Numéro(s) de document de la (des) demande(s)
initiale(s) en application de l'article 76 CBE:
**21819925.5 / 4 025 178**

(71) Demandeur: **Pierre Fabre Dermo-Cosmétique
92100 Boulogne-Billancourt (FR)**

(72) Inventeurs:
• **DROMIGNY, Hélène
31400 TOULOUSE (FR)**

• **TOUITOU, Karine
31770 COLOMIERS (FR)**
• **PROVOST, Roxane
31000 TOULOUSE (FR)**
• **PLANTIE, Marie
31120 LACROIX FALGARDE (FR)**

(74) Mandataire: **Regimbeau
20, rue de Chazelles
75847 Paris Cedex 17 (FR)**

Remarques:
Cette demande a été déposée le 30-11-2022 comme demande divisionnaire de la demande mentionnée sous le code INID 62.

(54) **NOUVEAU SYSTEME SOLUBILISANT DE FILTRES SOLAIRES ORGANIQUES LIPOSOLUBLES**

(57)     La présente invention a trait à une composition cosmétique ou dermatologique, sous forme d'émulsion, comprenant :

(a) un système photoprotecteur liposoluble, consistant en un ou plusieurs filtre(s) UV liposoluble(s) choisi(s) parmi :
(i) l'hexyl 2-[4-(diéthylamino)-2-hydroxybenzoyl]benzoate,
(ii) la 2,4-bis[4-(2-éthylhexyloxy)-2-hydroxyphényl]-6-(4-méthoxyphényl)-1,3,5-triazine,
(iii) le butyl méthoxydibenzoylméthane,
(iv) l'éthylhexyl triazone, et
(v) la diéthylhexyl butamido triazone ;

(b) un système solubilisant dudit système photoprotecteur liposoluble (a), comprenant du C12-C15 alkyl benzoate dans une quantité supérieure ou égale à 80 % en poids, de préférence supérieure ou égale à 90 % en poids, par rapport au poids total du système solubilisant ; et
(c) un ou plusieurs filtre(s) UV organique(s) particulaire(s), en particulier hydrodispersés, choisi(s) parmi :
(vi) la 5,6,5',6'-tétraphényl-3,3'-(1,4-phénylène)-bis[1,2,4]triazine,
(vii) le méthylène bis-benzotriazolyl tétraméthylbutylphenol, et
(viii) la tris-biphényl triazine ;

caractérisée en ce que :
- le système photoprotecteur liposoluble (a) représente entre 3 % et 19 % en poids, de préférence entre 5 % et 15 % en poids, par rapport au poids total de la composition ; et
- le système solubilisant (b) représente entre 15 % et 30 % en poids, par rapport au poids total de la composition ; ladite composition ne comprenant pas d'éthylhexyl salicylate, d'octocrylène, d'éthylhexyl méthoxycinnamate, d'isoamyl méthoxylcinnamate, d'homosalate, d'acide para-aminobenzoïque (PABA), d'octyl diméthyl PABA, de 3-méthylbenzylidène camphor, de 4-méthylbenzylidène camphor, de benzophénone-3 et/ou de benzophénone-4.

**Description**

Domaine de l'invention

La présente invention concerne une composition cosmétique ou dermatologique sous forme d'émulsion comprenant un système photoprotecteur liposoluble, un système solubilisant dudit système photoprotecteur liposoluble, et un ou plusieurs filtre(s) UV organique(s) particulaire(s), en particulier hydrodispersés.

Etat de la technique

Les effets délétères des rayonnements ultraviolets sur la peau sont bien connus, et une multitude de produits de protection solaire, communément appelés produits solaires, sont commercialisés. Ces produits solaires, destinés à une application topique, comprennent une association de composés organiques ou inorganiques, liposolubles, hydrosolubles ou non solubles, qui protègent l'utilisateur des rayonnements UV, et sont de ce fait qualifiés de filtres solaires.

La nécessité de combiner plusieurs filtres solaires au sein d'une même composition s'explique par le fait que chaque filtre absorbe de manière préférentielle certaines longueurs d'onde. Dès lors, afin de protéger efficacement l'utilisateur vis-à-vis de l'ensemble des rayonnements ultraviolets, il est nécessaire d'associer plusieurs filtres, pour obtenir un spectre d'absorption aux UV le plus large possible.

Toutefois, certains filtres solaires liquides dont l'éthylhexyl salicylate ou l'octocrylène sont connus pour faciliter la solubilisation des filtres organiques liposolubles, filtres solaires les plus utilisés dans les produits de protection solaire, dont nombre se présentent initialement sous forme de poudre.

Or, l'octocrylène, de même que l'éthylhexyl salicylate, sont suspectés d'être de potentiels perturbateurs endocriniens. D'autres filtres solaires présents dans nombre de produits commerciaux sont également sujet à controverse, en raison de leurs effets potentiellement néfastes pour l'utilisateur ou pour l'environnement. On peut notamment citer des dérivés de cinnamate (éthylhexyl méthoxycinnamate, isoamyl méthoxylcinnamate), d'homosalate, d'acide para-aminobenzoïque (PABA, octyl diméthyl PABA), du camphre (3-méthylbenzylidène camphor, 4-méthylbenzylidène camphor) ou de benzophénone (benzophénone-3, benzophénone-4). L'utilisation de tels composés n'est donc pas souhaitable.

Par ailleurs, de manière plus générale, il est souhaitable de minimiser d'une part le nombre de filtres ultraviolets constitutifs d'une composition photoprotectrice, et, d'autre part, la concentration desdits filtres au sein de la composition, afin d'obtenir de meilleurs résultats en termes de tolérance pour l'utilisateur et d'impact environnemental. En effet, non seulement les filtres chimiques organiques ne sont pas tous, en tant que tels, d'une parfaite innocuité, mais le risque d'interactions nuisibles entre les différents composés associés ne peut être ignoré. La minimisation du nombre et de la concentration des filtres au sein des produits solaires est en outre bénéfique pour l'environnement, dans la mesure où les filtres UV contenus dans un écran solaire peuvent se retrouver dans l'océan.

Par conséquent, la Demanderesse, qui place la protection de l'utilisateur et de l'environnement au centre de ses préoccupations dans le développement de nouveaux produits solaires, s'est interdit de recourir à l'utilisation de filtres solaires dont l'innocuité est mise en doute.

Il n'en demeure pas moins qu'il est nécessaire de parfaitement solubiliser les différents filtres constitutifs de la composition solaire, en particulier les filtres liposolubles se présentant sous forme de poudre, pour obtenir une composition homogène et stable dans le temps, de sorte à garantir une protection efficace vis-à-vis des rayonnements UV.

Outre sa sécurité, la Demanderesse a souhaité assurer le confort de l'utilisateur, en proposant un produit solaire à la sensorialité optimale, qui soit notamment facile à étaler, et qui, une fois appliqué, ne confère pas un aspect gras ou collant à la peau, que ce soit au toucher ou au visuel.

Problème technique

La Demanderesse a donc cherché à mettre au point de nouvelles compositions antisolaires protégeant efficacement l'utilisateur des rayonnements UV, comprenant un nouveau système solubilisant qui permet de solubiliser efficacement les filtres UV liposolubles sous forme de poudre, tout en présentant une sensorialité optimale. Il est à noter que la notion d'efficacité du système solubilisant recouvre celle de la stabilité de la solution obtenue par la solubilisation des filtres UV liposolubles dans le système solubilisant, au sein de laquelle les filtres ne doivent pas recristalliser au cours du temps.

De façon surprenante et inattendue, la Demanderesse a mis en évidence une nouvelle utilisation du $C_{12}$-$C_{15}$ alkyl benzoate comme principal solubilisant de filtres UV liposolubles dans une composition sous forme d'émulsion, qui apporte les propriétés recherchées en termes de protection solaire tout en offrant une galénique sensoriellement améliorée.

Par ailleurs, contrairement à la plupart des produits solaires actuellement sur le marché, la composition développée par la Demanderesse se distingue par de très bons résultats obtenus lors de tests de tolérance oculaire *in vitro.* Cela constitue un avantage considérable, puisqu'il est fréquent que les compositions solaires entrent en contact avec les yeux d'un utilisateur, du fait de la transpiration ou consécutivement à une baignade.

Résumé de l'invention

Selon un premier aspect, la présente invention concerne donc une composition cosmétique ou dermatologique sous forme d'émulsion comprenant :

**(a)** un système photoprotecteur liposoluble, consistant en un ou plusieurs filtre(s) UV liposoluble(s) choisi(s) parmi :

(i) l'hexyl 2-[4-(diéthylamino)-2-hydroxybenzoyl]benzoate,
(ii) la 2,4-bis[4-(2-éthylhexyloxy)-2-hydroxyphényl]-6-(4-méthoxyphényl)-1,3,5-triazine,
(iii) le butyl méthoxydibenzoylméthane,
(iv) l'éthylhexyl triazone, et
(v) la diéthylhexyl butamido triazone ;

**(b)** du $C_{12}$-$C_{15}$ alkyl benzoate ; et
**(c)** un ou plusieurs filtre(s) UV organique(s) particulaire(s), en particulier hydrodispersés, choisi(s) parmi :

(vi) la 5,6,5',6'-tétraphényl-3,3'-(1,4-phénylène)-bis[1,2,4]triazine,
(vii) le méthylène bis-benzotriazolyl tétraméthylbutylphenol, et
(viii) la tris-biphényl triazine ;

et est caractérisée en ce que :

- le système photoprotecteur liposoluble (a) représente entre 3 % et 19 %, notamment entre 5 % et 19 %, en particulier entre 7 % et 17 %, typiquement entre 9 % et 15 %, par exemple entre 10 % et 13 % en poids de la composition par rapport au poids total de la composition ; et
- le $C_{12}$-$C_{15}$ alkyl benzoate (b) représente entre 15 % et 30 % en poids, notamment entre 20 % et 30 % en poids, typiquement entre 20 % et 25 % en poids, par rapport au poids total de la composition ;

ladite composition ne comprenant pas d'éthylhexyl salicylate, d'octocrylène, d'éthylhexyl méthoxycinnamate, d'isoamyl méthoxylcinnamate, d'homosalate, d'acide para-aminobenzoïque (PABA), d'octyl diméthyl PABA, de 3-méthylbenzylidène camphor, de 4-méthylbenzylidène camphor, de benzophénone-3 et/ou de benzophénone-4.

Selon un second aspect, la présente invention concerne une composition cosmétique ou dermatologique sous forme d'émulsion, comprenant :

**(a)** un système photoprotecteur liposoluble, consistant en un ou plusieurs filtre(s) UV liposoluble(s) choisi(s) parmi les filtres (i) à (v) ;
**(b)** un système solubilisant dudit système photoprotecteur liposoluble (a), comprenant du $C_{12}$-$C_{15}$ alkyl benzoate dans une quantité supérieure ou égale à 80 % en poids, de préférence supérieure ou égale à 90 % en poids, par rapport au poids total du système solubilisant ; et
**(c)** un ou plusieurs filtre(s) UV organique(s) particulaire(s), en particulier hydrodispersés, choisi(s) parmi les filtres (vi) à (viii) ;

caractérisée en ce que :

- le système photoprotecteur liposoluble (a) représente entre 3 % et 19 % en poids, de préférence entre 5 % et 15 % en poids, par rapport au poids total de la composition ; et
- le système solubilisant (b) représente entre 15 % et 30 % en poids, par rapport au poids total de la composition ;

ladite composition ne comprenant pas d'éthylhexyl salicylate, d'octocrylène, d'éthylhexyl méthoxycinnamate, d'isoamyl méthoxylcinnamate, d'homosalate, d'acide para-aminobenzoïque (PABA), d'octyl diméthyl PABA, de 3-méthylbenzylidène camphor, de 4-méthylbenzylidène camphor, de benzophénone-3 et/ou de benzophénone-4.

Description détaillée

Par « émulsion », on entend, au sens de la présente invention, tout type d'émulsion obtenue par la dispersion d'une phase interne discontinue dans une phase externe continue, l'une de ces phases étant une phase aqueuse et l'autre étant une phase huileuse. Il s'agit, par exemple, d'une émulsion huile-dans-eau ou eau-dans-huile ou multiple, de préférence d'une émulsion huile-dans-eau.

Ces émulsions peuvent être plus ou moins fluides et se présenter notamment sous la forme d'une crème blanche ou colorée, d'une pommade, d'un lait, d'une lotion, d'un sérum, d'une pâte, ou éventuellement d'un aérosol, d'une mousse ou d'un spray, et peuvent également être résistantes à l'eau.

La composition selon l'invention est destinée à une application topique, notamment sur la peau et/ou les cheveux.

Par « système photoprotecteur », on entend, au sens de la présente invention, une association de composés qui permet, après application sur une surface (peau, cheveux, etc.), par des mécanismes d'absorption et/ou de réflexion et/ou de diffusion du rayonnement UVA et/ou UVB, d'empêcher, ou du moins de limiter, la mise en contact dudit rayonnement avec ladite surface. Les composés constituant le système photoprotecteur sont appelés des filtres solaires ou filtres UV.

Le terme « rayonnement ultraviolet » ou « rayonnement UV », communément abrégé « UV », désigne le rayonnement ultraviolet solaire mais également le rayonnement ultraviolet artificiel, généré par des lampes de bronzage par exemple.

Par « rayonnement UVA » ou simplement « UVA », on entend désigner les rayons ultraviolets ayant une longueur d'onde $\lambda$ comprise entre 320 et 400 nm.

Par « rayonnement UVB » ou simplement « UVB », on entend désigner les rayons ultraviolets ayant une longueur d'onde $\lambda$ comprise entre 290 et 320 nm.

Le terme « filtre solaire » ou « filtre UV » désigne une substance capable de filtrer les rayonnements UV pour protéger une surface, typiquement la peau ou les cheveux, contre les effets nocifs de ces radiations.

Un filtre solaire est qualifié de « filtre UVA » ou « filtre UVB » selon qu'il filtre majoritairement les UVA ou les UVB.

Un filtre solaire dit « large bande » ou encore « large spectre » filtre à la fois les UVA et les UVB.

Selon le mécanisme de filtration du rayonnement, c'est-à-dire selon que le rayonnement est absorbé et/ou réfléchi, voire diffusé par le filtre solaire, on distingue, en toute rigueur :

- les filtres *stricto sensu,* qui majoritairement absorbent le rayonnement, et
- les écrans, qui absorbent et réfléchissent le rayonnement.

Toutefois, dans la suite de la description, on utilisera indifféremment le terme « filtre solaire », parfois abrégé « filtre », qu'il s'agisse d'un filtre ou d'un écran.

Un filtre solaire peut être « liposoluble » ou « hydrosoluble », selon qu'il se solubilise plus facilement dans les lipides ou dans l'eau, ou encore « non soluble ». Un filtre solaire non soluble se présente typiquement sous forme particulaire, et peut néanmoins être intégré dans une composition de protection solaire, notamment sous une forme dispersée, en particulier hydrodispersée lorsqu'il est dispersé dans une phase aqueuse.

Le système photoprotecteur liposoluble **(a)** constitutif d'une composition selon l'invention comprend uniquement des filtres solaires de type liposoluble, et représente entre 3 % et 19 % en poids, de préférence entre 5 % et 15 % en poids, par rapport au poids total de la composition.

Dans un mode de réalisation particulier, le système photoprotecteur liposoluble représente entre 3 % et 19 %, notamment entre 5 % et 19 %, en particulier entre 7 % et 17 %, typiquement entre 9 % et 15 %, par exemple entre 10 % et 13 % en poids, par rapport au poids total de la composition.

Le système photoprotecteur liposoluble constitutif d'une composition selon l'invention consiste en un ou plusieurs filtre(s) UV liposoluble(s) choisi(s) parmi les filtres (i) à (v) suivants :

**(i)** hexyl 2-[4-(diéthylamino)-2-hydroxybenzoyl]benzoate (n° CAS : 302776-68-7), également appelé diéthylamino hydroxybenzoyl hexyl benzoate ou DHHB, commercialisé par BASF sous le nom d'Uvinul A Plus®.

Il s'agit d'un filtre UVA liposoluble, ayant une longueur d'onde d'absorbtion maximale $\lambda_{max}$ égale à 354 nm.

Dans un mode de réalisation particulier, le DHHB représente entre 1 % et 10%, par exemple entre 3 % et 10 %, en particulier entre 4 % et 9 %, typiquement entre 5 % et 8 %, en particulier entre 5 % et 7 %, notamment entre 6 % et 7 % ou avantageusement entre 5 % et 6 % en poids, par rapport au poids total de la composition.

**(ii)** 2,4-bis[4-(2-éthylhexyloxy)-2-hydroxyphényl]-6-(4-méthoxyphényl)-1,3,5-triazine (n° CAS : 187393-00-6), également appelée bis-éthylhexyloxyphenol méthoxyphenyl triazine ou BEMT, commercialisée par BASF sous le nom de Tinosorb S®.

Il s'agit d'un filtre large bande liposoluble, ayant deux pics d'absorption à 310 nm et 340 nm.

Dans un mode de réalisation particulier, la BEMT représente entre 1 % et 4 %, notamment entre 2 % et 4 %, en particulier entre 2 % et 3 % en poids par rapport au poids total de la composition.

**(iii)** 4-*tert*-butyl-4-méthoxydibenzoylmethane ou butyl méthoxydibenzoylméthane (nom chimique : 1,4-(1,1-diméthyléthyl)phényl-3-(4-méthoxyphényl)-1,3-propanedione ; n° CAS : 70356-09-1), également appelé avobenzone ou BMDBM, commercialisé notamment par DSM sous le nom de Parsol 1789®.

Il s'agit d'un filtre UVA liposoluble, ayant une longueur d'onde d'absorbtion maximale $\lambda_{max}$ égale à 357 nm.

Dans un mode de réalisation particulier, le BMDBM représente entre 1 % et 10 %, par exemple entre 1 % et 8 %, en particulier entre 1 % et 5 %, typiquement entre 2 % et 4 % en poids, par rapport au poids total de la composition.

**(iv)** éthylhexyl triazone ou EHT (nom chimique : acide 4-[[4,6-bis[[4-(2-éthylhexoxy-oxométhyl)phényl]amino]-1,3,5-triazin-2-yl]amino]benzoïque 2-éthylhexyl ester ; n° CAS : 88122-99-00), commercialisée par BASF sous le nom d'Uvinul T 150®.

Il s'agit d'un filtre UVB liposoluble, ayant une longueur d'onde d'absorbtion maximale $\lambda_{max}$ égale à 314 nm.

Dans un mode de réalisation particulier, l'EHT représente entre 1 % et 6 %, typiquement entre 2 % et 5 %, notamment entre 3 % et 5 %, en particulier entre 3,5 % et 4,5 % en poids par rapport au poids total de la composition.

**(v)** diéthylhexyl butamido triazone ou DBT (nom chimique : 4,4'-[[6-[[4-[[(1,1-diméthyléthyl)amino]carbonyl]phényl]amino]-1,3,5-triazine-2,4-diyl]diimino]bis-, bis(2-éthylhexyl)benzoate ; n° CAS : 154702-15-5), commercialisée par 3V Sigma sous le nom d'Uvasorb HEB®.

Il s'agit d'un filtre UVB liposoluble, ayant une longueur d'onde d'absorbtion maximale $\lambda_{max}$ égale à 310 nm.

Dans un mode de réalisation particulier, la DBT représente entre 1 % et 6 %, typiquement entre 2 % et 5 %, notamment entre 3 % et 5 %, en particulier entre 3,5 % et 4,5 % en poids par rapport au poids total de la composition.

Le système photoprotecteur liposoluble **(a)** peut consister en un unique filtre liposoluble choisi parmi les filtres (i) à (v).

Toutefois, de préférence, il consiste en l'association d'au moins deux filtres liposolubles, le premier étant choisi parmi les filtres (i) à (iii) et le second parmi les filtres (iv) et (v), le second filtre étant en particulier le filtre (iv). En particulier, le système photoprotecteur liposoluble **(a)** consiste en l'association de trois filtres, le premier étant choisi par les filtres (i) ou (iii), le second parmi les filtres (iv) et (v), et le troisième filtre correspondant au filtre (ii), le second filtre étant avantageusement le filtre (iv).

Dans un mode de réalisation particulier, le système photoprotecteur liposoluble **(a)** consiste en l'une des associations de filtres liposolubles suivantes :

**(a1)** hexyl 2-[4-(diéthylamino)-2-hydroxybenzoyl]benzoate, 2,4-bis[4-(2-éthylhexyloxy)-2-hydroxyphényl]-6-(4-méthoxyphényl)-1,3,5-triazine et éthylhexyl triazone ;

**(a2)** hexyl 2-[4-(diéthylamino)-2-hydroxybenzoyl]benzoate, 2,4-bis[4-(2-éthylhexyloxy)-2-hydroxyphényl]-6-(4-méthoxyphényl)-1,3,5-triazine et diéthylhexyl butamido triazone ;

**(a3)** 2,4-bis[4-(2-éthylhexyloxy)-2-hydroxyphényl]-6-(4-méthoxyphényl)-1,3,5-triazine et éthylhexyl triazone ;

**(a4)** 2,4-bis[4-(2-éthylhexyloxy)-2-hydroxyphényl]-6-(4-méthoxyphényl)-1,3,5-triazine et diéthylhexyl butamido triazone ;

**(a5)** hexyl 2-[4-(diéthylamino)-2-hydroxybenzoyl]benzoate, butyl méthoxydibenzoylméthane , 2,4-bis[4-(2-éthylhexyloxy)-2-hydroxyphényl]-6-(4-méthoxyphényl)-1,3,5-triazine et éthylhexyl triazone ;

**(a6)** hexyl 2-[4-(diéthylamino)-2-hydroxybenzoyl]benzoate, butyl méthoxydibenzoylméthane, 2,4-bis[4-(2-éthylhexyloxy)-2-hydroxyphényl]-6-(4-méthoxyphényl)-1,3,5-triazine et diéthylhexyl butamido triazone ;

**(a7)** butyl méthoxydibenzoylméthane , 2,4-bis[4-(2-éthylhexyloxy)-2-hydroxyphényl]-6-(4-méthoxyphényl)-1,3,5-triazine et éthylhexyl triazone ; et

**(a8)** butyl méthoxydibenzoylméthane, 2,4-bis[4-(2-éthylhexyloxy)-2-hydroxyphényl]-6-(4-méthoxyphényl)-1,3,5-triazine et diéthylhexyl butamido triazone.

En particulier, le système photoprotecteur liposoluble **(a)** consiste en l'une des associations (a1), (a3), (a5) ou (a7).

Lorsque le système photoprotecteur liposoluble **(a)** correspond à l'une des associations de filtres liposolubles (a1) - (a8), celle-ci représente entre 3 % et 19 % en poids, de préférence entre 5 % et 15 % en poids, par rapport au poids total de la composition.

Dans un mode de réalisation particulier, le système photoprotecteur liposoluble correspond à l'une des associations de filtres liposolubles (a1) - (a8), laquelle représente entre 3 % et 19 %, notamment entre 5 % et 19 %, en particulier entre 7 % et 17 %, typiquement entre 9 % et 15 %, par exemple entre 10 % et 13 % en poids de la composition par rapport au poids total de la composition.

En particulier, le système photoprotecteur liposoluble **(a)** consiste en l'association (a1) ou (a2).

Dans ce mode de réalisation particulier, le DHHB représente entre 1 % et 10%, par exemple entre 3 % et 10 %, en particulier entre 4 % et 9 %, typiquement entre 5 % et 8 %, par exemple entre 5 % et 7 %, notamment entre 6 % et 7 % ou avantageusement entre 5 % et 6 % en poids, par rapport au poids total de la composition, la BEMT représente entre 1 % et 4 %, notamment entre 2 % et 4 %, en particulier entre 2 % et 3 % en poids de la composition par rapport au poids total de la composition, et l'EHT ou la DBT représente entre 1 % et 6 %, typiquement entre 2 % et 5 %, notamment entre 3 % et 5 %, en particulier entre 3,5 % et 4,5 % en poids par rapport au poids total de la composition.

Dans un mode de réalisation avantageux, le système photoprotecteur liposoluble **(a)** consiste en l'association de filtres liposolubles **(a1)**.

Le système photoprotecteur liposoluble **(a)** est solubilisé dans un système solubilisant **(b)**.

Au sens de la présente invention, le terme « système solubilisant », étant entendu du système photoprotecteur liposoluble **(a)** tel que décrit précédemment, désigne un composé lipophile liquide ou une combinaison de plusieurs composé(s) lipophile(s) liquide(s), typiquement un émollient lipophile liquide ou une combinaison d'émollient(s) lipophile(s) liquide(s), notamment un corps gras liquide ou une combinaison de corps gras liquide(s), apte à solubiliser totalement le ou les filtre(s) UV liposoluble(s) (i) à (v), permettant ainsi d'obtenir une solution homogène transparente, le ou les filtre(s) UV liposoluble(s) (i) à (v) étant solubilisé(s) dans une ou des quantité(s) telle(s) que le ratio pondéral du ou des filtre(s) UV liposoluble(s) (i) à (v) / système solubilisant est supérieur ou égal à 0,2, typiquement au moins égal à 0,3, de préférence au moins égal à 0,4, et notamment inférieur à 0,7, en particulier inférieur ou égal à 0,6.

Par « composé lipophile liquide », on entend désigner un composé qui présente une affinité pour les substances apolaires tels que les lipides, et se présente sous forme liquide notamment pour une température comprise entre 5°C et la température ambiante.

Par « température ambiante », on entend, au sens de la présente invention, une température comprise entre 15°C et 25°C, de préférence entre 20°C et 25°C.

Par « solution homogène », on entend désigner l'absence de cristaux et/ou agrégats visibles à l'œil nu dans la solution.

Par « solution transparente », on entend désigner une solution limpide, c'est-à-dire qui ne présente aucun trouble.

Cette aptitude à solubiliser le ou les filtre(s) UV liposoluble(s) (i) à (v) est évaluée en deux temps.

Dans un premier temps, le ou les filtre(s) UV liposoluble(s) (i) à (v) sont introduits dans le système solubilisant (b) à la température de préparation de la phase huileuse de l'émulsion selon l'invention, typiquement à 80° C, sous agitation, de sorte à le ou les solubiliser.

Une solution homogène limpide doit être obtenue dans une durée inférieure à 5 heures, notamment inférieure à 4 heures, en particulier inférieure à 2 heures, et de préférence inférieure à 1 heure. La solution homogène transparente ainsi obtenue est ensuite refroidie jusqu'à température ambiante, ou jusqu'à 5°C, en présence ou en l'absence d'agitation.

Après solubilisation, typiquement réalisée dans les conditions décrites ci-dessus, le système solubilisant selon l'invention permet de maintenir le ou les filtre(s) UV liposoluble(s) (i) à (v) sous forme solubilisée, c'est-à-dire qu'il prévient la recristallisation du ou des filtre(s) UV liposoluble(s) (i) à (v), même partielle.

Ainsi, une fois la solution homogène obtenue par la solubilisation du ou des filtre(s) UV liposoluble(s) (i) à (v) dans le système solubilisant (b), celle-ci demeure homogène et limpide, en présence ou en l'absence d'agitation, à une température de 5°C et à température ambiante, pendant au moins 24h, de préférence au moins 1 semaine, en particulier au moins 1 mois.

Dans un mode de réalisation préféré, la solution obtenue par la solubilisation du ou des filtre(s) UV liposoluble(s) (i) à (v) dans le système solubilisant (b) demeure homogène et limpide, en présence ou en l'absence d'agitation, à température ambiante, et avantageusement à une température de 5°C, pendant au moins 6 mois, de préférence au moins 12 mois, notamment au moins 24 mois, en particulier au moins 36 mois.

Le système solubilisant **(b)** selon l'invention comprend du $C_{12}$-$C_{15}$ alkyl benzoate dans une quantité supérieure ou égale à 80 % en poids, de préférence supérieure ou égale à 90 % en poids, avantageusement supérieure ou égale à 95 % en poids, notamment supérieure ou égale à 98 % en poids, en particulier supérieure ou égale à 99 % en poids, par rapport au poids total du système solubilisant.

Selon un mode de réalisation particulier, le $C_{12}$-$C_{15}$ alkyl benzoate représente 100 % en poids par rapport au poids total du système solubilisant.

Par « $C_{12}$-$C_{15}$ alkyl benzoate », on entend, au sens de la présente invention, un composé de formule générale $C_6H_5$-C(O)OR, dans laquelle R représente un groupement ($C_{12}$-$C_{15}$)-alkyle, ou un mélange de ceux-ci.

Par groupement « ($C_{12}$-$C_{15}$)alkyle », on entend, au sens de la présente invention, une chaîne hydrocarbonée monovalente saturée, linéaire ou ramifiée, comportant 12 à 15 atomes de carbone.

Le terme « $C_{12}$-$C_{15}$ alkyl benzoate » renvoie notamment aux produits commerciaux suivants : Cetiol AB[®] (BASF), Crodamol AB[®] (Croda), Finsolv TN[®] (Innospec) et Tegosoft TN[®] (Evonik).

Comme cela ressort de ce qui précède, l'homme du métier pourra vérifier que la combinaison d'un ou de plusieurs composé(s) lipophile(s) liquide(s), typiquement d'un ou de plusieurs émollient(s) lipophile(s) liquide(s), avec du $C_{12}$-$C_{15}$ alkyl benzoate comme composant majoritaire, lequel représente au moins 80 %, de préférence au moins 90 %, avantageusement au moins 95 %, notamment au moins 98 %, en particulier au moins 99 %, notamment 100 % en poids par rapport au poids total de ladite combinaison, constitue un système solubilisant au sens de la présente invention, en appliquant le protocole suivant :

- introduire le système photoprotecteur liposoluble selon l'invention, tel que décrit précédemment, dans le système solubilisant, dans un ratio pondéral système liposoluble / système solubilisant supérieur ou égal à 0,2, typiquement au moins égal à 0,3, de préférence au moins égal à 0,4, et notamment inférieur à 0,7, en particulier inférieur ou égal à 0,6 ;
- placer le mélange obtenu sous agitation, de préférence sous agitation mécanique au moyen d'un barreau aimanté, à une température comprise entre 70°C et 85°C, typiquement à 80°C, et le maintenir sous agitation pendant une durée comprise entre 5 min et 5h, notamment entre 10 min et 4h, en particulier entre 20 min et 2h, typiquement entre 30 min et 1h ;
- constater l'obtention d'une solution homogène transparente, c'est-à-dire l'absence de trouble et de cristaux et/ou agrégats visibles à l'œil nu dans la solution ;
- refroidir la solution homogène à température ambiante, c'est-à-dire typiquement à une température comprise entre 15°C et 25°C, de préférence entre 20° C et 25°C, en présence ou en l'absence d'agitation ;
- laisser la solution reposer en l'état pendant au moins 24h, notamment au moins 1 semaine et jusqu'à 3 mois, typiquement pendant 1 mois ; et
- constater le maintien de l'homogénéité de la solution, c'est-à-dire l'absence de cristaux et/ou agrégats visibles à l'œil nu dans la solution.

Alternativement, après obtention d'une solution homogène transparente, la solution peut être refroidie jusqu'à environ 5°C, typiquement à 5°C, et être laissée au repos en l'absence d'agitation pendant au moins 24h, notamment au moins 1 semaine et jusqu'à 1 mois, pour ensuite constater le maintien de l'homogénéité de la solution.

Le ou les composé(s) lipophile(s) liquide(s) pouvant être combiné(s) au $C_{12}$-$C_{15}$ alkyl benzoate pour former le système solubilisant selon l'invention comportent typiquement une ou plusieurs fonction(s) chimique(s) polaire(s) choisis parmi les fonctions ester (-$CO_2$-), alcool (-OH), éther (-O-), carbonate (-$OCO_2$-) et acide carboxylique (-$CO_2H$), et un ou plusieurs groupement(s) lipophile(s), c'est-à-dire un ou plusieurs groupement(s) hydrocarboné(s) aliphatiques et/ou aromatique(s), en particulier une ou plusieurs chaîne(s) hydrocarbonée(s) saturée(s) ou insaturée(s), linéaire(s) ou ramifiée(s), comportant entre 6 et 20 atomes de carbone, par exemple entre 7 et 18 atomes de carbone, notamment entre 8 et 16 atomes de carbone, un groupement phényle (-$C_6H_5$) et/ou un groupement phénylalkyle.

Dans un mode de réalisation particulier, le ou les composé(s) lipophile(s), notamment le ou les émollient(s) lipophile(s) liquide(s) pouvant être combiné(s) au $C_{12}$-$C_{15}$ alkyl benzoate pour former le système solubilisant selon l'invention, sont choisi(s) parmi les triglycérides caprylique/caprique, le dicaprylyl carbonate, le coco-caprylate, le diisopropyl sébacate, le diéthylhexyl succinate, l'isopropyl palmitate, l'isopropyl myristate, le propylheptyl caprylate, le phénéthyl benzoate, le dibutyl adipate, le diisopropyl adipate, le glycéryl tri-2-éthylhexanoate, le pentaerythrityl tetra-2-éthylhexanoate, le cétyl 2-éthylhexanoate, l'isononyl isonanoate, le butylène glycol dicaprylate/dicaprate, l'octyldodécanol, le propylène glycol dicaprylate/dicaprate et le dicaprylyl éther, en particulier parmi les triglycérides caprylique/caprique, le dicaprylyl carbonate, l'isopropyl palmitate, le propylheptyl caprylate, le phénéthyl benzoate, le diisopropyl adipate, le glycéryl tri-2-éthylhexanoate, le pentaerythrityl tetra-2-éthylhexanoate, le cétyl 2-éthylhexanoate, l'isononyl isononoate, le butylène glycol dicaprylate/dicaprate, l'octyldodécanol, le propylène glycol dicaprylate/dicaprate et le dicaprylyl éther.

Dans un mode de réalisation particulier, le système solubilisant (b) est constitué de un ou plusieurs émollient(s) lipophile(s) liquide(s) choisi(s) parmi le $C_{12}$-$C_{15}$ alkyl benzoate, les triglycérides caprylique/caprique et le dicaprylyl carbonate, de préférence parmi le $C_{12}$-$C_{15}$ alkyl benzoate et les triglycérides caprylique/caprique, le $C_{12}$-$C_{15}$ alkyl ben-zoate représentant une quantité supérieure ou égale à 80 % en poids, de préférence supérieure ou égale à 90 % en poids, avantageusement supérieure ou égale à 95 % en poids, notamment supérieure ou égale à 98 % en poids, en particulier supérieure ou égale à 99 % en poids, notamment 100 % en poids, par rapport au poids total du système solubilisant.

Le système solubilisant (b) représente entre 15 % et 30 % en poids, notamment entre 20 % et 30 % en poids, typiquement entre 20 % et 25 % en poids, par rapport au poids total de la composition.

En particulier, le $C_{12}$-$C_{15}$ alkyl benzoate représente entre 15 % et 30 % en poids, notamment entre 20 % et 30 % en poids, typiquement entre 20 % et 25 % en poids, par rapport au poids total de la composition.

Dans un mode de réalisation particulier d'une composition selon l'invention, le système photoprotecteur liposoluble **(a)** est tel que décrit précédemment, et le ratio pondéral système photoprotecteur liposoluble **(a)** / système solubilisant **(b)** est supérieur ou égal à 0,2, typiquement supérieur ou égal à 0,3, notamment supérieur ou égal à 0,4, avantageusement supérieur ou égal à 0,5, en particulier strictement supérieur à 0,5, et notamment inférieur à 0,7, typiquement inférieur ou égal à 0,6, en particulier inférieur ou égal à 0,55.

En particulier, le système photoprotecteur liposoluble **(a)** est tel que décrit précédemment, et le ratio pondéral système photoprotecteur liposoluble **(a)** / $C_{12}$-$C_{15}$ alkyl benzoate est supérieur ou égal à 0,2, typiquement supérieur ou égal à 0,3, notamment supérieur ou égal à 0,4, avantageusement supérieur ou égal à 0,5, en particulier strictement supérieur à 0,5, et notamment inférieur à 0,7, typiquement inférieur ou égal à 0,6, en particulier inférieur ou égal à 0,55.

Le système photoprotecteur liposoluble **(a)** représente entre 3 % et 19 %, notamment entre 5 % et 19 %, en particulier entre 7 % et 17 %, typiquement entre 9 % et 15 %, par exemple entre 10 % et 13 % en poids de la composition par rapport au poids total de la composition, et consiste en particulier en l'une des associations (a1)-(a8), notamment en l'une des associations (a1), (a3), (a5) ou (a7), avantageusement en l'association (a1).

Le système solubilisant **(b)** représente entre 15 % et 30 % en poids, notamment entre 20 % et 30 % en poids, typiquement entre 20 % et 25 % en poids, par rapport au poids total de la composition, et est en particulier constitué de un ou plusieurs émollient(s) lipophile(s) liquide(s) choisi (s) parmi le $C_{12}$-$C_{15}$ alkyl benzoate, les triglycérides caprylique/caprique et le dicaprylyl carbonate, de préférence parmi le $C_{12}$-$C_{15}$ alkyl benzoate et les triglycérides caprylique/caprique, le $C_{12}$-$C_{15}$ alkyl benzoate représentant une quantité supérieure ou égale à 80 % en poids, de préférence supérieure ou égale à 90 % en poids, avantageusement supérieure ou égale à 95 % en poids, notamment supérieure ou égale à 98 % en poids, en particulier supérieure ou égale à 99 % en poids, notamment 100 % en poids, par rapport au poids total du système solubilisant.

Dans un mode de réalisation particulier, la composition selon l'invention ne comprend pas de diisopropyl sébacate, diéthylhexyl succinate, isopropyl myristate et/ou dibutyl adipate.

Dans un autre mode de réalisation particulier, la composition selon l'invention ne comprend pas de dicaprylyl carbonate, coco-caprylate, diisopropyl sébacate, diéthylhexyl succinate, isopropyl palmitate, isopropyl myristate, propylheptyl caprylate, phénéthyl benzoate, dibutyl adipate, diisopropyl adipate, glycéryl tri-2-éthylhexanoate, pentaerythrityl tetra-2-éthylhexanoate, cétyl 2-éthylhexanoate, isononyl isononoate, butylène glycol, dicaprylate/dicaprate, octyldodécanol, propylène glycol dicaprylate/dicaprate et/ou dicaprylyl éther.

La composition selon l'invention comprend en outre (c) un ou plusieurs filtre(s) UV organique(s) particulaire(s), en particulier hydrodispersés, choisi(s) parmi les filtres (vi) à (viii) suivants.

**(vi)** 5,6,5',6'-tétraphényl-3,3'-(1,4-phénylène)-bis[1,2,4]triazine, (n° CAS : 55514-22-2) également appelée phénylène bis-diphényltriazine.

Il s'agit d'un filtre essentiellement UVA, ayant une longueur d'onde d'absorption maximale $\lambda_{max}$ égale à 355 nm, de type non soluble. Il s'agit en particulier d'un filtre hydrodispersible.

La phénylène bis-diphényltriazine se présente typiquement sous la forme d'une dispersion aqueuse, comprenant entre 20 % et 50 %, en particulier entre 40 % et 50 % en poids de matière active par rapport au poids totale de la dispersion.

Avantageusement, le procédé de préparation de la dispersion aqueuse consiste en un broyage du filtre organique insoluble sous forme de particules, à l'aide d'un appareil de broyage et en présence d'un adjuvant de broyage.

Classiquement, des procédés de broyage en voie humide sont utilisés (broyage humide, malaxage par voie humide) et l'adjuvant de broyage permet une meilleure dispersion des particules. Ces techniques sont bien connues de l'homme de l'art.

L'appareil de broyage peut être par exemple un broyeur à microbilles, un broyeur vibrant ou un broyeur à boulets.

En fonction du procédé de broyage, l'adjuvant de broyage est choisi dans le groupe constitué des tensioactifs anioniques, non ioniques ou amphotères, des émulsifiants et des dispersants tel que le PPG-1-PEG-9 Lauryl Glycol Ether (Eumulgin® L, commercialisée par BASF).

Dans un mode de réalisation particulier, la phénylène bis-diphényltriazine représente entre 1 % et 5 %, notamment entre 2 % et 5 %, en particulier entre 2 % et 4 %, typiquement entre 3 % et 4 % en poids par rapport au poids total de la composition.

Il est entendu que ces pourcentages massiques, ainsi que l'ensemble de ceux indiqués dans la description, sont exprimés en pourcentage de masse de matière active par rapport à la masse total de la composition.

Dans un mode de réalisation particulier, la taille $D_{50}$ des particules de phénylène bis-diphényltriazine est comprise entre 100 et 1000 nm, plus particulièrement entre 100 et 500 nm, encore plus particulièrement entre 120 et 400 nm, notamment entre 120 et 250 nm, en particulier entre 120 et 200 nm, typiquement entre 150 et 200 nm.

Par « taille $D_{50}$ » ou « médiane », on entend désigner la taille pour laquelle la fonction cumulative F(D) est égale à 50 %, F(D) étant définie selon la relation suivante :

$$F(D_i) = \int_0^{D_i} f(D)\,dD$$

dans laquelle :

f(D) est la distribution de tailles des particules en nombre, et $D_i$ est une classe de taille.

La valeur de la $D_{50}$ sera déterminée selon la méthode décrite dans les exemples de la présente description.

**(vii)** méthylène bis-benzotriazolyl tétraméthylbutylphénol (nom chimique : 2,2'-méthylène-bis-(6-(2H-benzotriazol-2-yl)-4-(1,1,3,3-tétraméthylbutyl)phénol ; n° CAS : 103597-45-1) également appelé MBBT, commercialisé par BASF sous le nom de Tinosorb M®.

Il s'agit d'un filtre large bande, ayant deux pics d'absorption à 306 nm et 348 nm, de type non soluble.

Le MBBT se présente typiquement sous la forme d'une dispersion aqueuse, comprenant entre 40 % et 60 %, typiquement 50 % en poids de matière active par rapport au poids totale de la dispersion.

Dans un mode de réalisation particulier, le MBBT représente entre 1 % et 7 %, typiquement 2 % et 6 %, notamment entre 2 % et 5 %, en particulier entre 3 % et 4 % en poids par rapport au poids total de la composition.

Dans un mode de réalisation particulier, la taille $D_{50}$ des particules de MBBT est comprise entre 50 nm et 250 nm, en particulier entre 60 nm et 150 nm.

**(viii)** 2,4,6-tris(biphényl-4-yl)-1,3,5-triazine (n° CAS : 31274-51-8) également tris-biphényl triazine ou TBPT, commercialisé par BASF sous le nom de Tinosorb A2B®.

Il s'agit d'un filtre essentiellement UVB, ayant une longueur d'onde d'absorption maximale $\lambda_{max}$ égale à 310 nm, de type non soluble.

La TBPT se présente typiquement sous la forme d'une dispersion aqueuse, comprenant entre 40 % et 60 %, typiquement 50 % en poids de matière active par rapport au poids totale de la dispersion.

Dans un mode de réalisation particulier, la TBPT représente entre 1 % et 7 %, en particulier entre 2 % et 7 %, notamment entre 2 % et 6 % en poids par rapport au poids total de la composition.

Dans un mode de réalisation particulier, la taille $D_{50}$ des particules de TBPT est comprise entre 50 nm et 250 nm, en particulier entre 80 nm et 150 nm.

Dans un mode de réalisation particulier de la composition selon l'invention, le ou les filtre(s) UV organique(s) particulaire(s), en particulier hydrodispersés, sont choisi(s) parmi la phénylène bis-diphényltriazine (vi) et le MBBT (vii).

Avantageusement, la composition selon l'invention comprend un unique filtre UV organique particulaire, de préférence la phénylène bis-diphényltriazine.

Selon un mode de réalisation particulier de la composition selon l'invention, le ratio pondéral filtre(s) UV organique(s) particulaire(s) (c) / système photoprotecteur liposoluble (a) est inférieur ou égal à 0,35, en particulier inférieur ou égal à 0,33, avantageusement inférieur ou égal à 0,32, notamment inférieur ou égal à 0,30, et notamment supérieur ou égal à 0,2.

Selon un mode de réalisation particulier, la composition cosmétique ou dermatologique selon l'invention comprend :

(a) un système photoprotecteur liposoluble, consistant en un ou plusieurs filtre(s) UV liposoluble(s) choisi(s) parmi les filtres (i) à (v) ;

(b) un système solubilisant dudit système photoprotecteur liposoluble (a), comprenant du $C_{12}$-$C_{15}$ alkyl benzoate dans une quantité supérieure ou égale à 80 % en poids, de préférence supérieure ou égale à 90 % en poids, par rapport au poids total du système solubilisant ; et

(c) la phénylène bis-diphényltriazine en tant que filtre UV organique particulaire hydrodispersé, avantageusement dans une quantité comprise entre 1 % et 5 %, notamment entre 2 % et 5 %, en particulier entre 2 % et 4 %, typiquement entre 3 % et 4 % en poids par rapport au poids total de la composition,

et est caractérisée en ce que :

- le système photoprotecteur liposoluble (a) représente entre 3 % et 19 %, notamment entre 5 % et 19 %, en particulier entre 7 % et 17 %, typiquement entre 9 % et 15 %, par exemple entre 10 % et 13 % en poids de la composition par rapport au poids total de la composition ; et
- le système solubilisant (b) représente entre 15 % et 30 % en poids, par rapport au poids total de la composition ;

ladite composition ne comprenant pas d'éthylhexyl salicylate, d'octocrylène, d'éthylhexyl méthoxycinnamate, d'isoamyl méthoxylcinnamate, d'homosalate, d'acide para-aminobenzoïque (PABA), d'octyl diméthyl PABA, de 3-méthylbenzylidène camphor, de 4-méthylbenzylidène camphor, de benzophénone-3 et/ou de benzophénone-4.

Dans ce mode de réalisation particulier, le système photoprotecteur liposoluble (a) et son système solubilisant (b) sont tels que décrits précédemment, de même que le ratio pondéral (a) / (b).

Avantageusement, le ratio pondéral phénylène bis-diphényltriazine (c) / système photoprotecteur liposoluble (a) est inférieur ou égal à 0,35, en particulier inférieur ou égal à 0,33, avantageusement inférieur ou égal à 0,32, notamment inférieur ou égal à 0,30, et notamment supérieur ou égal à 0,2.

Avantageusement, le ratio pondéral phénylène bis-diphényltriazine **(c)** / système photoprotecteur liposoluble **(a)** est inférieur ou égal à 0,35, en particulier inférieur ou égal à 0,33, avantageusement inférieur ou égal à 0,32, notamment inférieur ou égal à 0,30, et notamment supérieur ou égal à 0,2, et le ratio pondéral système photoprotecteur liposoluble **(a)** / $C_{12}$-$C_{15}$ alkyl benzoate est supérieur ou égal à 0,2, typiquement supérieur ou égal à 0,3, notamment supérieur ou égal à 0,4, avantageusement supérieur ou égal à 0,5, en particulier strictement supérieur à 0,5 et notamment inférieur à 0,7, typiquement inférieur ou égal à 0,6, en particulier inférieur ou égal à 0,55.

Dans un mode de réalisation particulier, le système photoprotecteur liposoluble **(a)** correspond à l'une des associations de filtres liposolubles (a1) - (a8), laquelle représente entre 3 % et 19 %, notamment entre 5 % et 19 %, en particulier entre 7 % et 17 %, typiquement entre 9 % et 15 %, par exemple entre 10 % et 13 % en poids de la composition par rapport au poids total de la composition.

En particulier, le système photoprotecteur liposoluble **(a)** consiste en l'association (a1).

Dans ce mode de réalisation particulier, le DHHB représente entre 1 % et 10%, par exemple entre 3 % et 10 %, en particulier entre 4 % et 9 %, typiquement entre 5 % et 8 %, par exemple entre 5 % et 7 %, notamment entre 6 % et 7 % ou avantageusement entre 5 % et 6 % en poids, par rapport au poids total de la composition, la BEMT représente entre 1 % et 4 %, notamment entre 2 % et 4 %, en particulier entre 2 % et 3 % en poids de la composition par rapport au poids total de la composition, et l'EHT représente entre 1 % et 6 %, typiquement entre 2 % et 5 %, notamment entre 3 % et 5 %, en particulier entre 3,5 % et 4,5 % en poids par rapport au poids total de la composition.

Dans un mode de réalisation particulier, la composition selon l'invention ne comprend pas de diisopropyl sébacate, diéthylhexyl succinate, isopropyl myristate et/ou dibutyl adipate.

Dans un autre mode de réalisation particulier , la composition selon l'invention ne comprend pas de dicaprylyl carbonate, cooc-caprylate, diisopropyl sébacate, diéthylhexyl succinate, isopropyl palmitate, isopropyl myristate, propylheptyl caprylate, phénéthyl benzoate, dibutyl adipate, diisopropyl adipate, glycéryl tri-2-éthylhexanoate, pentaerythrityl tetra-2-éthylhexanoate, cétyl 2-éthylhexanoate, isononyl isononoate, butylène glycol dicaprylate/dicaprate, octyldodécanol, propylène glycol dicaprylate/dicaprate et/ou dicaprylyl éther.

Dans un mode de réalisation particulier, la composition selon l'invention ne comprend aucun filtre hydrosoluble.

L'absence de filtres hydrosolubles dans une composition selon l'invention est particulièrement avantageuse, en cela que les filtres hydrosolubles sont, de manière générale, plus facilement assimilables par les organismes marins que les filtres liposolubles ou non solubles. Par conséquent, le choix de la Demanderesse consistant à recourir uniquement à des filtres liposolubles ou particulaires, notamment hydrodispersés, permet de minimiser l'impact de la composition selon l'invention sur le milieu marin.

Dans un mode de réalisation particulier, la composition selon l'invention, comprend un ou plusieurs filtre(s) inorganique(s) particulaire(s), tel que du dioxyde de titane ($TiO_2$) ou de l'oxyde de zinc (ZnO).

Le ou les filtres inorganique(s) particulaire(s) éventuellement présent dans la composition selon l'invention ont typiquement une taille $D_{50}$ de particule comprise entre 15 nm et 150 nm.

Dans un autre mode de de réalisation particulier, la composition selon l'invention ne comprend aucun filtre inorganique particulaire.

Dans un mode de réalisation particulier, le nombre total de filtres UV présents dans la composition est inférieur ou égal à 5, de préférence inférieur ou égal à 4, et l'ensemble des filtres UV présents dans la composition représentent entre 4 % et 25 % en poids, en particulier entre 6 % et 20 % en poids, notamment entre 7 % et 18 % en poids, typiquement entre 8 % et 17 % en poids, par rapport au poids total de la composition.

Avantageusement, la composition selon l'invention est photostable.

Par « photostable », on entend au sens de la présente invention, qu'après une irradiation de 5 DEM et de préférence 10 DEM, on conserve au minimum 80%, de préférence au minimum 85 % et avantageusement au minimum 90 % du FPS total.

Le terme « FPS » désigne le facteur de protection solaire, communément appelé sous son acronyme anglais SPF (« *sun protection factor* »), le FPS total étant évalué pour les longueurs d'onde comprises entre 290 à 400 nm.

Sa valeur sera déterminée selon la méthode décrite dans les exemples de la présente description recommandée par le COLIPA (Fédération Européenne des Industries de la Parfumerie), dans ses directives diffusées en mars 2011.

Dans un mode de réalisation particulier, le FPS d'une composition selon l'invention est supérieur ou égal à 50, avantageusement supérieur ou égal à 60.

Dans un autre mode de réalisation particulier, le ratio FPS/UVA est inférieur ou égal à 3, conformément à la règlementation en vigueur. Le dénominateur dudit quotient correspond à la part UVA (320 à 400 nm) du FPS total, et peut être calculé selon la méthode recommandée par le COLIPA (Fédération Européenne des Industries de la Parfumerie), dans ses directives diffusées en mars 2011. Avantageusement, le ratio FPS/UVA est inférieur ou égal à 2,5.

La composition selon l'invention peut en outre comprendre un ou plusieurs excipient(s) cosmétiquement ou dermatologiquement acceptable(s).

Dans la présente invention, on entend désigner par « cosmétiquement ou dermatologiquement acceptable » ce qui est utile dans la préparation d'une composition cosmétique ou dermatologique qui est généralement sûr, non toxique et ni biologiquement ni autrement non souhaitable et qui est acceptable pour une utilisation cosmétique ou dermatologique humaine.

Par « excipient cosmétiquement ou dermatologiquement acceptable », on entend tout adjuvant cosmétiquement ou dermatologiquement acceptable permettant la fabrication, la conservation ou l'administration de la composition.

Les compositions selon l'invention peuvent ainsi comprendre des adjuvants pharmaceutiques ou dermatologiques classiques notamment choisis parmi les émulsifiants, les agents de consistance, les agents de rémanence à l'eau, les agents de texture, les opacifiants, les pigments colorants, les agents anti-mousse, les parfums, les conservateurs, les polymères, les charges, les séquestrants, les bactéricides, les absorbeurs d'odeur, les agents alcalinisants ou acidifiants, les tensioactifs, les antiradicaux libres, les vitamines E et C, les alpha-hydroxyacides, des agents actifs (en particulier des adoucissants, des agents hydratants, des antioxydants) ou tout autre ingrédient habituellement utilisé pour la fabrication de compositions antisolaires.

La composition peut comprendre en outre un polyol miscible à l'eau à la température ambiante (environ 25°C), notamment choisi parmi les polyols ayant notamment de 2 à 20 atomes de carbones, de préférence ayant de 2 à 10 atomes de carbone, et préférentiellement ayant de 2 à 6 atomes de carbones, tels que la glycérine ; les dérivés de glycol tel que le propylène glycol, le butylène glycol, le pentylène glycol, l'hexylène glycol, le dipropylène glycol, le diéthylène glycol ; les éthers de glycol tels que les éthers d'alkyle en $C_1$-$C_4$ de mono-, di- ou tri-propylène glycol, les éthers d'alkyle en $C_1$-$C_4$ de mono-, di- ou tri-éthylène glycol et leurs mélanges.

La composition peut également comprendre ou un plusieurs émulsifiant(s) anioniques, cationiques et non ioniques choisi(s) parmi les dérivés du glucose tels que le cétéaryl glucoside, l'arachidyl glucoside, le lauryl glucoside, le polyglycéryl-3 méthylglucose distéarate et le méthyl glucose sesquistearate ; les dérivés du saccharose tels que le polystéarate de saccharose et le palmitate de saccharose ; les glycérides d'acides gras tels que le stéarate de glycéryle, l'oléate de glycéryle et le glycéryl stéarate citrate ; les dérivés de l'acide glutamique tel que le stéaroyl glutamate de sodium ; les dérivés de l'acide sulfosuccinique tel que le cétéaryl-sulfosuccinate disodique et le sulfosuccinate de dioctyle de sodium ; les dérivés de l'acide phosphorique tel que le cétyl phosphate de potassium ; les esters d'acides gras de polyglycéryle tels que le polyglycéryl-3-diisostéarate et le polyglycéryl-2-dipolyhydroxystéarate ; les dérivés du sorbitol tels que le polysorbate-n, PEG-10 sorbitan laurate et les esters de sorbitane ; les polyglycoléthers d'alcool gras et d'acide gras tels que le cétéareth-20, le beheneth-25, le stéareth-2, le trideceth-9, le PEG-5 éthylhexanoate et le PEG-100 stéarate ; le sulfate de lauryle de sodium, le stéarate de sodium, et les copolymères et dérivés de silicone / polysiloxane / polyalkyle / polyéther oxyalcénylés organomodifiés.

Dans un mode de réalisation particulier, le ou les émulsifiant (s) représente(nt) entre 1 % et 5 %, de préférence entre 1 % et 3 % en poids, par rapport au poids total de la composition.

Dans un mode de réalisation particulier, la composition selon l'invention comprend un ou plusieurs émulsifiant (s) choisi(s) parmi le cétyl phosphate de potassium, le lauryl glucoside et le glycéryl stéarate citrate.

Dans un mode de réalisation particulier, la composition selon l'invention ne comprend pas d'émulsifiant éthoxylé et/ou comprenant de chaîne PEG.

En particulier, la composition ne comprend pas de dérivés du sorbitol tels que le polysorbate-n et le PEG-10 sorbitan laurate ; de polyglycoléthers d'alcool gras et d'acide gras tels que le cétéareth-20, le beheneth-25, le stéareth-2 et le PEG-100 stéarate ; et de copolymères et dérivés de silicone / polysiloxane / polyalkyle / polyéther oxyalcénylés organomodifiés.

La composition peut également comprendre ou un plusieurs agent(s) de consistance sous forme solide choisi (s) parmi les alcools gras tels que l'alcool cétylique, l'alcool cétéarylique et l'alcool stéarylique ; les acides gras tel que l'acide stéarique ; les esters d'acides gras tel que le stéarate de myristyle ; les polysaccharides ou dérivés tels que la gomme xanthane, la gomme guar, la gomme agar, les alginates, la gomme gellane et le carraghénane ; les dérivés de la cellulose tel que l'hydroxypropylcellulose ; les cires telles que la cire d'abeille, la cire de carnauba, la cire microcristalline, la cérésine, et l'ozocérite ; les polyacrylates ou homopolymères d'acides acryliques ou de polyacrylamides réticulés tels que les carbomères, les copolymères d'acrylate, les copolymères réticulés acrylate/($C_{10}$-$C_{30}$)alkylacrylate, les copolymères acrylate/méthacrylate de béhénéthyle-25 ; et les dérivés de silicates tels que les silicates de magnésium.

Dans un mode de réalisation particulier, le ou les agent(s) de consistance représente(nt) entre 0,1 % et 2 %, de préférence entre 0,1 % et 1 % en poids, par rapport au poids total de la composition.

Dans un mode de réalisation particulier, la composition selon l'invention comprend un ou plusieurs agent(s) de consistance choisi (s) parmi les alcools gras tels que l'alcool cétylique, l'alcool cétéarylique et l'alcool stéarylique ; les acides gras tel que l'acide stéarique ; les esters d'acides gras tel que le stéarate de myristyle ; les polysaccharides ou dérivés tels que la gomme xanthane, la gomme guar, la gomme agar, les alginates, la gomme gellane et le carraghénane ; et les dérivés de la cellulose tel que l'hydroxypropylcellulose.

Dans un mode de réalisation particulier, la composition selon l'invention ne comprend pas d'agent de consistance choisi parmi les acrylates et leurs dérivés.

La composition peut également comprendre ou un plusieurs agent(s) de rémanence à l'eau choisi(s) parmi un copolymère de N-vinylpyrrolidone (VP) et d'eicosène (INCI : VP/eicosen copolymer), un polymère réticulé de triméthylpentanediol, d'acide adipique et de glycérol (INCI : trimethylpentanediol/adipic acid/glycerin crosspolymer), le polyamide 3 (INCI), un copolymère d'acrylate et d'octylacrylamide (INCI : acrylates/octylacrylamide copolymer), et le triacontanyl PVP (INCI).

Dans un mode de réalisation particulier, le ou les agent(s) de rémanence à l'eau représente(nt) entre 0 % et 2 %, de préférence entre 0 % et 1 % en poids, par rapport au poids total de la composition.

De préférence, la composition selon l'invention comprend du VP / eicosène copolymère en tant qu'agent de rémanence à l'eau.

La composition peut également comprendre ou un plusieurs agent(s) de texture sous forme de poudre(s), en particulier choisi(s) parmi les ingrédients de la famille des amidons, tels que les amidons de riz, de maïs, et du tapioca ; des talcs et des dérivés de celluloses tel que la cellulose microcristalline.

Dans un mode de réalisation particulier, le ou les agent(s) de texture représente(nt) entre 1 % et 10 %, de préférence entre 1 % et 5 % en poids, par rapport au poids total de la composition.

Dans un mode de réalisation particulier, la composition selon l'invention ne comprend pas d'agent de texture choisi parmi les dérivés du nylon et le polyméthacrylate de méthyle (PMMA).

La composition peut également comprendre des pigments colorants, en particulier choisis parmi le CI77891, le CI77491, le CI77492 et le CI77499 (désignations INCI).

Dans un mode de réalisation particulier, la composition selon l'invention comprend une quantité d'alcools en $C_1$-$C_4$, tels que l'éthanol et l'isopropanol, inférieure ou égale à 4 % en poids, de préférence inférieure ou égale à 2 % en poids, notamment inférieure ou égale à 1 % en poids, en particulier inférieure ou égale à 0,5 % en poids, par rapport au poids total de la composition.

Dans un autre mode de réalisation particulier, la composition selon l'invention est dépourvue d'éthanol et d'isopropanol.

Dans un mode de réalisation particulier, la composition selon l'invention comprend une quantité d'ingrédients volatils inférieure ou égale à 5 % en poids, de préférence inférieure ou égale à 2 % en poids, notamment inférieure ou égale à 1 % en poids, en particulier inférieure ou égale à 0,5 % en poids, par rapport au poids total de la composition.

Par « ingrédients volatils », on entend, au sens de la présente invention, les composés organiques ou inorganiques volatils non alcooliques tels que des esters, des silicones ou des alcanes volatils, caractérisés par un point éclair inférieur à 60° C.

Par « point éclair » (noté PE), on entend, au sens de la présente invention, la température minimale à laquelle une substance combustible émet des vapeurs en concentration suffisante pour former avec l'air ambiant un mélange gazeux qui s'enflamme au contact d'une flamme ou d'un point chaud, mais insuffisante pour que la combustion se propage d'elle-même en l'absence de la flamme dite pilote. Selon le règlement (CE) N° 1272/2008 relatif à la classification, à l'étiquetage et à l'emballage des substances et des mélanges, une substance ayant un PE tel que 23°C ≤ PE < 60°C appartient à la catégorie 3 « faiblement inflammable ».

Parmi les esters volatils, on peut notamment citer l'isohexyl neopentanoate.

Parmi les silicones volatils, on peut notamment citer le cyclopentasiloxane.

Parmi les alcanes volatils, on peut notamment citer l'isohexadecane.

Dans un autre mode de réalisation particulier, la composition selon l'invention est dépourvue d'ingrédients volatils au sens de la présente invention.

La composition peut également comprendre des agents alcalinisants ou acidifiants, et plus particulièrement des acides et des bases permettant d'ajuster la zone de pH de ladite composition. Les bases peuvent être minérales (soude, potasse, ammoniaque, etc.) ou organiques, telles que la mono-, di- ou triéthanolamine, un aminométhylpropanediol, la N-méthyl-glucamine, les acides aminés basiques comme l'arginine et la lysine, et leurs mélanges. Les acides peuvent être minéraux (acide chlorhydrique, etc.) ou organiques, tels que l'acide lactique et l'acide citrique.

Les compositions selon l'invention peuvent comprendre en outre des agents actifs additionnels choisis notamment parmi les agents hydratants, les agents desquamants, les agents améliorant la fonction barrière, les agents dépigmentants, les agents antioxydants, les agents dermocontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les inhibiteurs de NO synthase, les agents augmentant l'activité de la glande sébacée, les agents tenseurs, les agents liporestructurants, les agents amincissants, les agents favorisant la microcirculation cutanée, les agents apaisants et/ou irritants, les séborégulateurs ou anti-séborrhéiques, les agents astringents, les agents cicatrisants, les agents antiinflammatoires, les agents anti-acné, et leurs mélanges.

Dans un mode de réalisation particulier, la composition selon l'invention est dépourvue de silicone.

La composition selon l'invention est plus particulièrement une composition de protection solaire, communément appelée composition solaire, destinée à la protection de la peau (visage et/ou corps) et/ou des cheveux contre les rayonnements ultraviolets.

La présente invention a également pour objet une méthode de protection de la peau (visage et/ou corps) et/ou des cheveux contre les rayonnements ultraviolets, comprenant l'application sur la peau (visage et/ou corps) et/ou sur les cheveux d'une composition décrite précédemment.

La présente invention concerne en outre une composition telle que précédemment décrite pour son utilisation pour la protection de la peau (visage et/ou corps) et/ou des cheveux contre les rayonnements ultraviolets.

La présente invention concerne de surcroît l'utilisation d'une composition telle que précédemment décrite pour la protection de la peau (visage et/ou corps) et/ou des cheveux contre les rayonnements ultraviolets.

La présente invention concerne par ailleurs un procédé de préparation d'une composition selon l'invention telle que décrite précédemment, sous forme d'émulsion huile dans eau, comprenant les étapes suivantes :

- solubilisation du système de filtre(s) liposoluble(s) (a) dans le système solubilisant (b) à une température comprise entre 70°C et 85°C, typiquement à 80°C, sous agitation, de préférence sous agitation mécanique ;
- ajout des autres ingrédients constitutifs de la phase huileuse de l'émulsion au mélange obtenu précédemment, à une température comprise entre 70°C et 85°C, typiquement à 80°C, sous agitation, de préférence sous agitation mécanique ;
- dispersion du ou des filtre(s) organique(s) particulaire(s) (c) dans de l'eau ; à une température comprise entre 70°C et 85°C, typiquement à 80°C, sous agitation, de préférence sous agitation mécanique ;
- ajout des autres ingrédients constitutifs de la phase aqueuse de l'émulsion au mélange obtenu précédemment, à une température comprise entre 70°C et 85°C, typiquement à 80°C, sous agitation, de préférence sous agitation mécanique ;
- introduction de la phase huileuse précédemment obtenue dans la phase aqueuse précédemment obtenue, sous agitation, de préférence sous agitation mécanique, jusqu'à obtenir une solution homogène ;
- refroidissement de l'émulsion ainsi obtenue jusqu'à température ambiante ; et
- optionnellement, ajout de parfum, d'agent(s) actifs et/ou d'agent(s) de texture sous forme de poudre(s) sous agitation, de préférence sous agitation mécanique.

## EXEMPLES

Les abréviations suivantes ont été utilisées :

| DEM | : dose érythémateuse minimale |
| --- | --- |
| FPS | : facteur de protection solaire |
| MTT | : bromure de 3-(4,5-diméthylthiazol-2-yl)-2,5-diphényltétrazolium |
| PEG | : polyéthylène glycol |
| PMMA | : polyméthylméthacrylate |
| UV | : rayonnement ultra-violet |
| VP | : N-vinylpyrrolidone |

## I. Système solubilisant

L'aptitude de trois combinaisons S1, S2 et S3 d'émollients lipophiles liquides à solubiliser le même système photoprotecteur liposoluble, en l'espèce le système (a1) tel que décrit précédemment, a été évaluée selon le protocole suivant :

- Solubilisation du système photoprotecteur liposoluble (a1) à 80°C sous agitation, de préférence sous agitation mécanique au moyen d'un barreau aimanté, dans la combinaison d'émollients lipophiles ;
- Refroidissement à température ambiante ;
- Stockage d'un échantillon de la solution obtenue à température ambiante, et refroidissement d'un autre échantillon à 5°C, pour stockage également ;
- Observation visuelle régulière dans le temps de l'apparition d'une éventuelle recristallisation.

Les résultats obtenus pour les combinaisons S1, S2 et S3 sont reproduits dans le tableau ci-dessous, dans lequel la quantité de chaque émollient lipophile est exprimée en pourcentage massique par rapport au poids total de la combinaison S1, S2 ou S3, et la quantité de chaque filtre liposoluble est exprimée en pourcentage massique par rapport au poids total du système photoprotecteur liposoluble (a1).

| Filtres liposolubles (% m/m) | Système photoprotecteur liposoluble (a1) | | |
|---|---|---|---|
| BEMT | 20 - 25 | | |
| EHT | 30 - 35 | | |
| DHHB | 45 - 50 | | |
| **Emollients lipophiles** (% m/m) | **S1** | **S2** | **S3** |
| dicaprylyl carbonate | - | 39 | 36 |
| triglycérides caprylique/caprique | - | 9 | 16 |
| $C_{12}$-$C_{15}$ alkyl benzoate | 100 | 52 | 4 |
| **Ratio système photoprotecteur liposoluble (a1) / émollient(s) lipophile(s)** | 0,61 | 0,53 | 0,48 |
| **Observation visuelle** | | | |
| 24h à température ambiante | Aucun cristal | Aucun cristal | Aucun cristal |
| 1 mois à 5°C | Aucun cristal | Quelques cristaux | Quelques cristaux |

Seul S1 constitue un système solubilisant selon la présente invention, qui permet d'une part de solubiliser le système photoprotecteur liposoluble de sorte à obtenir une solution homogène limpide, et d'autre part de prévenir la recristallisation des filtres UV liposolubles dans le temps.

A titre comparatif, la stabilité d'une autre phase huileuse d'une émulsion de protection solaire, correspondant à celle de l'exemple B3 de la demande WO 2016/012586, a été évaluée selon le protocole suivant :

- Solubilisation du système photoprotecteur liposoluble à 80°C sous agitation mécanique au moyen d'un barreau aimanté dans le système solubilisant ;
- Refroidissement à température ambiante ;
- Stockage de la solution obtenue à température ambiante ;
- Observation visuelle régulière dans le temps de l'apparition d'une éventuelle recristallisation.

| Système photoprotecteur liposoluble (a) | |
|---|---|
| BMDBM | 5 g soit 23 % (m/m) |
| DHHB | 16,7 g soit 77 % (m/m) |
| **Système solubilisant (b)** | |
| $C_{12}$-$C_{15}$ alkyl benzoate | 13,3 g (100 %) |
| **Ratio (a) / (b)** | 1,63 |
| **Observation visuelle** | |
| 24h à température ambiante | Aucun cristal |
| 15 jours à température ambiante | Très nombreux cristaux |

## II. Compositions

Dans les formules qui suivent, la 5,6,5',6'-tetraphenyl-3,3'-(1,4-phénylène)-bis[1,2,4]triazine est sous la forme d'une dispersion aqueuse comprenant 45 % (m/m) en poids de matière active et est caractérisée par une $D_{50}$ comprise entre 150 et 250 nm.

### II.1. Procédé de préparation des émulsions

Les ingrédients de la phase huileuse (en particulier le(s) filtre(s) UV liposoluble(s), le système solubilisant, le(s) émulsifiant(s), le ou les agent(s) de consistance liposoluble(s)) d'une part, et les ingrédients de la phase aqueuse (en particulier le(s) filtre(s) organique(s) particulaire(s), le(s) polyol(s), le ou les agent(s) de consistance hydrosoluble(s), et l'eau) d'autre part, sont respectivement mélangés et chauffés à 80°C sous agitation.

Puis la phase huileuse est ajoutée à la phase aqueuse sous agitation jusqu'à homogénéisation.

Enfin, la composition ainsi obtenue est refroidie jusqu'à température ambiante sous agitation.

Optionnellement, le parfum, le(s) agent(s) actifs ainsi que le ou les agent(s) de texture sous forme de poudre(s) sont ajoutés sous agitation après refroidissement de la composition finale.

**11.2. Formule 1**

| Ingrédients | % (m/m) |
|---|---|
| **Système photoprotecteur liposoluble** | |
| Bis-éthylhexyloxyphenol méthoxyphényl triazine | 2-3 |
| Diéthylamino hydroxybenzoyl hexyl benzoate | 5-7 |
| Ethyl hexyl triazone | 3-4 |
| **Système solubilisant** | |
| $C_{12}$-$C_{15}$ alkyl benzoate | 20-25 |
| **Filtre organique particulaire** | |
| 5,6,5',6'-tetraphenyl-3,3'-(1,4-phénylène)-bis[1,2,4]triazine | 3-4 |
| **Autres composants** | |
| Glycérine | 4,0 |
| Eau déminéralisée | qsp 100 |
| Gomme xanthane | 0,2 |
| Conservateurs | qs |
| Parfum | qs |
| Alcool stéarylique | 1,5 |
| Monostéarate de glycérol | 2,0 |
| VP / eicosène copolymère | 1,0 |
| Amidon de riz | 2-5 |
| Cétyl phosphate de potassium | 2,0 |

Le système photoprotecteur liposoluble représente avantageusement entre 12 % et 14 % en poids par rapport au poids total de la composition.

**II.3. Formule 2**

| Ingrédients | % (m/m) |
|---|---|
| **Système photoprotecteur liposoluble** | |
| Bis-éthylhexyloxyphenol méthoxyphényl triazine | 2-3 |
| Diéthylamino hydroxybenzoyl hexyl benzoate | 5-7 |
| Ethyl hexyl triazone | 3-4 |

(suite)

| Système solubilisant | |
|---|---|
| C$_{12}$-C$_{15}$ alkyl benzoate | 20-25 |
| **Filtre organique particulaire** | |
| 5,6,5',6'-tetraphenyl-3,3'-(1,4-phénylène)-bis[1,2,4]triazine | 3-4 |
| **Autres composants** | |
| Glycérine | 3,0 |
| Eau déminéralisée | qsp 100 |
| Gomme xanthane | 0,3 |
| Conservateurs | qs |
| Parfum | qs |
| Alcool stéarylique | 1,0 |
| Monostéarate de glycérol | 1,0 |
| VP / eicosène copolymère | 1,0 |
| Amidon de riz | 2-5 |
| Cétyl phosphate de potassium | 2,0 |

Le système photoprotecteur liposoluble représente avantageusement entre 12 % et 14 % en poids par rapport au poids total de la composition.

**II.4. Formule 3**

| Ingrédients | % (m/m) |
|---|---|
| **Système photoprotecteur liposoluble** | |
| Bis-éthylhexyloxyphenol méthoxyphényl triazine | 2-3 |
| Diéthylamino hydroxybenzoyl hexyl benzoate | 5-7 |
| Ethyl hexyl triazone | 3-4 |
| **Système solubilisant** | |
| C$_{12}$-C$_{15}$ alkyl benzoate | 20-25 |
| Triglycérides caprylique/caprique | 0,2 |
| **Filtre organique particulaire** | |
| Méthylène bis-benzotriazolyl tétraméthylbutylphenol | 2-4 |
| **Autres composants** | |
| Glycérine | 4,0 |
| Eau déminéralisée | qsp 100 |
| Gomme xanthane | 0,2 |
| Conservateurs | qs |
| Parfum | qs |
| Alcool stéarylique | 1,5 |
| Monostéarate de glycérol | 2,0 |

(suite)

| Autres composants | |
|---|---|
| VP / eicosène copolymère | 1,0 |
| Cétyl phosphate de potassium | 2,0 |

**II.5. Formule 4**

| Ingrédients | % (m/m) |
|---|---|
| **Système photoprotecteur liposoluble** | |
| Bis-éthylhexyloxyphenol méthoxyphényl triazine | 2-3 |
| Diéthylamino hydroxybenzoyl hexyl benzoate | 6-7 |
| Diéthylhexyl butamido triazone | 3-4 |
| **Système solubilisant** | |
| $C_{12}$-$C_{15}$ alkyl benzoate | 20-25 |
| **Filtre organique particulaire** | |
| 5,6,5',6'-tetraphenyl-3,3'-(1,4-phénylène)-bis[1,2,4]triazine | 3-4 |
| **Autres composants** | |
| Glycérine | 4,0 |
| Eau déminéralisée | qsp 100 |
| Gomme xanthane | 0,2 |
| Conservateurs | qs |
| Alcool cétylique | 1,5 |
| Monostéarate de glycérol | 2,0 |
| VP / eicosène copolymère | 1,0 |
| Lauryl glucoside | 5,0 |

**II.6. Formule 5**

| Ingrédients | % (m/m) |
|---|---|
| **Système photoprotecteur liposoluble** | |
| Bis-éthylhexyloxyphenol méthoxyphényl triazine | 2-3 |
| Butyl méthoxydibenzoylméthane | 3-4 |
| Ethyl hexyl triazone | 3-4 |
| **Système solubilisant** | |
| $C_{12}$-$C_{15}$ alkyl benzoate | 20-25 |
| **Filtre organique particulaire** | |
| 5,6,5',6'-tetraphenyl-3,3'-(1,4-phénylène)-bis[1,2,4]triazine | 3-4 |

(suite)

| Autres composants | |
|---|---|
| Glycérine | 4,0 |
| Eau déminéralisée | qsp 100 |
| Gomme xanthane | 0,2 |
| Conservateurs | qs |
| Parfum | qs |
| Alcool stéarylique | 1,5 |
| Monostéarate de glycérol | 2,0 |
| VP / eicosène copolymère | 1,0 |
| Amidon de riz | 2-5 |
| Potassium cétyl phosphate | 2,0 |

**II.7. Formule 6**

| Ingrédients | % (m/m) |
|---|---|
| **Système photoprotecteur liposoluble** | |
| Diéthylamino hydroxybenzoyl hexyl benzoate | 5-6 |
| Ethyl hexyl triazone | 3-4 |
| **Système solubilisant** | |
| $C_{12}$-$C_{15}$ alkyl benzoate | 20-25 |
| **Filtre organique particulaire** | |
| 5,6,5',6'-tetraphenyl-3,3'-(1,4-phénylène)-bis[1,2,4]triazine | 3-4 |
| méthylène bis-benzotriazolyl tétraméthylbutylphenol | 5-7 |
| **Autres composants** | |
| Glycérine | 4,0 |
| Eau déminéralisée | qsp 100 |
| Gomme xanthane | 0,2 |
| Conservateurs | qs |
| Parfum | qs |
| Alcool stéarylique | 1,5 |
| Monostéarate de glycérol | 2,0 |
| VP / eicosène copolymère | 1,0 |
| Cellulose microcristalline | 2-5 |
| Cétyl phosphate de potassium | 2,0 |

**II.8. Formule 7**

| Ingrédients | % (m/m) |
|---|---|
| **Système photoprotecteur liposoluble** | |
| Bis-éthylhexyloxyphenol méthoxyphényl triazine | 3-4 |
| Diéthylamino hydroxybenzoyl hexyl benzoate | 5-6 |
| **Système solubilisant** | |
| $C_{12}$-$C_{15}$ alkyl benzoate | 20-25 |
| **Filtre organique particulaire** | |
| 5,6,5',6'-tetraphenyl-3,3'-(1,4-phénylène)-bis[1,2,4]triazine | 3-4 |
| **Filtre minéral particulaire** | |
| $TiO_2$ | 4-5 |
| **Autres composants** | |
| Glycérine | 4,0 |
| Eau déminéralisée | qsp 100 |
| hydroxypropylcellulose | 0,2 |
| Conservateurs | qs |
| Parfum | qs |
| Alcool stéarylique | 1,5 |
| Monostéarate de glycérol | 2,0 |
| VP / eicosène copolymère | 1,0 |
| Amidon de maïs | 2-5 |
| Cétyl phosphate de potassium | 2,0 |

### III. Détermination de la taille des particules de 5,6,5',6'-tetraphenyl-3,3'-(1,4-phenylene)-bis[1,2,4]triazine

La $D_{50}$ des particules de phénylène bis-diphényltriazine est déterminée à l'aide d'un granulomètre à diffraction laser Mastersizer 3000 en voie liquide.

Un échantillon de dispersion aqueuse de phénylène bis-diphényltriazine est placé sous agitation magnétique (2500 tr/min) pendant 2 min.

1 mL de l'échantillon est prélevé et introduit dans une fiole que l'on complète à 100 mL avec de l'eau ultrapure.

La solution ainsi obtenue est placée sous agitation magnétique (2 500 tr/min) pendant 30 s. On prélève la solution à examiner pour l'introduire dans le vase de mesure jusqu'à la limite d'obscuration de 15 %.

3 essais indépendants sont réalisés et 3 mesures (laser rouge : mesure bruit de fond, 10 s, mesure de l'échantillon, 10 s ; laser bleu : mesure bruit de fond, 10 s, mesure de l'échantillon, 10 s) indépendantes sont effectuées par essai.

Les résultats sont obtenus en volume, et ensuite convertis en nombre à l'aide du logiciel.

### IV. Evaluation de la protection solaire

L'évaluation des paramètres permettant de quantifier la photoprotection assurée par une composition selon l'invention est réalisée selon les méthodes recommandées par le COLIPA (Fédération Européenne des Industries de la Parfumerie), dans ses directives diffusées en mars 2011.

**IV.1. Matériel**

*- Le spectromètre UV*

Le spectrophotomètre mesure la transmittance spectrale à travers une plaque avec et sans couche de composition anti-solaire sur sa surface.

Le spectrophotomètre doit permettre des mesures comprises entre 290 nm et 400 nm. Pour réduire la variabilité entre les lectures de mesure et pour compenser le manque d'uniformité dans la couche de produit, il est recommandé que l'aire des zones de lecture soit d'au moins 0,5 cm$^2$.

Le spectrophotomètre utilisé pour ces mesures est le Labsphere® UV-1000S ou 2000S.

*- La plaque*

La plaque est le matériel sur lequel est appliquée la composition anti-solaire. Ce matériel doit être transparent aux UV, non fluorescent, photostable et inerte par rapport aux composés des compositions testées. Pour ce protocole, les plaques de PMMA se sont avérées idéales.

*- Source UV*

La source UV est un simulateur solaire avec une lampe arc xenon diffusant un spectre Visible + UVA + UVB. La source UV utilisée pour cette étude est le Suntest CPS+ (Atlas).

**IV.2. Méthodes de détermination du FPS et de la photostabilité**

*- Mesures de la transmission ou travers d'une plaque non traitée*

Dans un premier temps, il est nécessaire de déterminer la transmission d'UV à travers la plaque témoin. Celle-ci est préparée en étalant quelques microlitres de glycérine de façon à ce que la surface de la plaque soit totalement recouverte.

*- Application de l'échantillon*

L'échantillon à tester est appliqué sur la plaque de PMMA à une dose de 1,3 mg/cm$^2$ (quantité réelle restant sur la plaque). Pour garantir l'exactitude de la dose et la reproductibilité des résultats, la zone d'application est supérieure à 10 cm$^2$. L'échantillon à tester est appliqué sous forme d'un grand nombre de petites gouttes de même volume, distribuées sur l'ensemble de la surface de la plaque.

Pour s'assurer que la quantité de produit est correcte, une méthode de validation de la quantité de produit appliqué doit être adoptée (par exemple : peser la plaque avant et après application du produit).

Après application de la quantité de l'échantillon définie, l'échantillon doit être étalé sur l'ensemble de la plaque aussi rapidement que possible (moins de 30 secondes).

L'échantillon est ensuite placé pendant 15 minutes dans l'obscurité à température ambiante afin de favoriser la formation d'un film homogène.

*- Mesure de la transmission ou travers d'une plaque traitée avec l'échantillon*

La plaque traitée avec l'échantillon est analysée au spectrophotomètre et la valeur moyenne de transmission des rayons UV à travers l'échantillon est déterminée pour chaque longueur d'onde de 290 nm à 400 nm (en utilisant les données d'absorbances monochromatiques mesurées sur les différentes zones de la plaque).

*- Nombre de mesures*

Il faut préparer au moins trois plaques de PMMA pour chaque échantillon. Chaque plaque doit être mesurée dans au moins neuf domaines différents sauf si presque toute la surface de la plaque est mesurée par spectrophotométrie.

*- Calcul du **FPS in vitro** (Facteur de Protection Solaire, communément désigné sous son acronyme anglais SPF)*

Il est effectué à partir des données d'absorbance A($\lambda$) avant et après irradiation aux doses de 5 et 10 DEM, selon la formule suivante :

$$FPS\ in\ vitro = \frac{\int_{\lambda=290\,nm}^{\lambda=400\,nm} E(\lambda).S(\lambda).d\lambda}{\int_{\lambda=290\,nm}^{\lambda=400\,nm} E(\lambda).S(\lambda).10^{-A(\lambda)}d\lambda}$$

dans laquelle :

E(λ) correspond au spectre d'efficacité érythémale,
S(λ) correspond à l'irradiance solaire spectrale,
A(λ) correspond à l'absorbance de l'échantillon,
dλ est la variation de longueur d'onde (1 nm).

- *Calcul de la **photostabilité***

$$Photostabilit\acute{e}\ UV\ totale = \frac{FPS\ apr\grave{e}s\ irradiation}{FPS\ avant\ irradiation}\ x\ 100$$

Les compositions selon l'invention peuvent être classées selon les catégories indiquées ci-après, en fonction de leur photostabilité.

| Catégorie | % du FPS total conservé après irradiation de 5 DEM |
|---|---|
| A | ≥ 90% |
| B | 85% - <90% |
| C | 80% - <85% |
| D | ≤ 80 % |

### IV.3. Résultats

Les résultats suivants ont été obtenus pour les formules 1 et 2 :

| | Formule 1 | Formule 2 |
|---|---|---|
| FPS *in vitro* | > 70 | > 70 |
| Ratio FPS / UVA | < 3 | < 3 |
| Photostabilité totale après irradiation à 5 DEM | Catégorie A | Catégorie A |

### V. Evaluation de la tolérance oculaire

### V.1. Modèle *in vitro* sur épithélium reconstruit de cornée humaine (HCE)

Ce test est une méthode alternative à l'expérimentation animale pour l'évaluation du potentiel irritant oculaire sur épithélium de cornée humaine reconstruit.

Le principe est basé sur la détermination de l'indice de cytotoxicité moyen sur 24 heures par application des produits sur des épithéliums de cornées humaines reconstruites.

La viabilité cellulaire est étudiée par réduction du MTT incorporé dans les cellules vivantes en sel de tétrazolium qui forme des cristaux violets mesurable par colorimétrie.

Le paramètre calculé est le $MCI_{24h}$ (« *mean cytotoxicity index* » sur 24 heures) soit la moyenne de mortalité engendrée par heure. Celui-ci permet de déterminer si le produit est irritant ou non.

La méthode est décrite dans l'article Doucet et al., Toxicology in vitro, 2006, 20, 499-512.

Les compositions selon l'invention peuvent être classées selon le système de cotation indiqué ci-après, en fonction de leur $MCI_{24h}$.

| Catégorie | $MCI_{24h}$ |
|---|---|
| 4 : non irritant | < 4 |
| 3 : légèrement irritant | 4 - < 7 |

(suite)

| Catégorie | $MCI_{24h}$ |
|---|---|
| 2 : moyennement irritant | 7 - < 17 |
| 1 : très irritant | ≥ 17 |

**V.2. Méthode « Relargage du Rouge Neutre » (RRN)**

Ce test est une méthode alternative à l'expérimentation animale pour l'évaluation du potentiel irritant oculaire.

Le principe est basé sur l'évaluation de la cytotoxicité par détermination de la concentration entrainant 50 % de mortalité (CL50) à l'aide de la technique de relargage rouge neutre sur fibroblastes de cornée de lapin de lignée SIRC après un temps de contact de 30 ou 60 secondes.

La méthode est décrite dans l'arrêté du 27 décembre 1999 publié au Journal Officiel français du 30 décembre 1999.

Après incubation de la solution colorante de rouge neutre avec les cellules en culture sur plaque chaque dilution de produit est mise en contact avec les cellules pendant 60 secondes ou 30 secondes, avant d'être éliminée par rinçage. La viabilité des cellules est évaluée par spectrophotométrie après ajout de la solution de révélation.

La cytotoxicité du produit est donnée par une échelle décrite dans le Journal Officiel français du 30 décembre 1999, comprenant 4 catégories :

| Catégorie | Cytotoxicité |
|---|---|
| 4 | négligeable |
| 3 | peu importante |
| 2 | modérée |
| 1 | importante |

**V.3. Résultats**

Les résultats suivants ont été obtenus pour la formule 1 :

| | Formule 1 |
|---|---|
| Modèle HCE | Cotation 3 |
| Méthode RRN | Cotation 4 |

**VI. Evaluation de la sensorialité d'une composition selon l'invention par un test d'usage consommateur**

Le but de ce test est de caractériser les perceptions et ressentis lors de l'utilisation d'une composition selon l'invention et d'en déterminer les forces et faiblesses dans des conditions réelles d'utilisation.

78 consommatrices ont été recrutées en France, elles sont âgées de 18 à 65 ans (moyenne 42 ans), et 84 % d'entre elles ont la peau sensible.

Ces consommatrices ont évalué le produit correspondant à la formule 2 chez elles.

Le produit a été évalué anonymement sur une période d'utilisation de 14 jours, avec une évaluation requise à l'application, au bout de 1 jour, et au bout de 14 jours.

A noter qu'aucune intolérance conduisant à l'arrêt du test n'a été relevée.

Le protocole d'utilisation indiqué aux consommatrices est le suivant.

Avant de sortir, appliquer la composition sur le cou et le visage en remplacement du produit solaire habituel.

Renouveler l'application fréquemment, plus particulièrement après avoir transpiré, nagé, ou essuyé la peau.

| Critère | Non gras | Non collant | Facilité d'application | Fini imperceptible | Invisible sur la peau |
|---|---|---|---|---|---|
| Note moyenne (/10) | 7,5 | 7,64 | 8,08 | 7,36 | 7,96 |
| % consommatrices ayant attribué une note comprise entre 7 et 10 | 77 % | 77 % | 82 % | 73 % | 86 % |

La composition de produit solaire selon l'invention présente bien une sensorialité optimale, laquelle s'est notamment traduite lors du test d'usage par un produit facile à étaler, et qui, une fois appliqué, ne confère pas un aspect gras ou collant à la peau. Un très bon ressenti des consommatrices au toucher et au visuel est également à noter.

**Revendications**

1. Composition cosmétique ou dermatologique, sous forme d'émulsion, comprenant :

   **(a)** un système photoprotecteur liposoluble, consistant en un ou plusieurs filtre(s) UV liposoluble(s) choisi(s) parmi :

      (i) l'hexyl 2-[4-(diéthylamino)-2-hydroxybenzoyl]benzoate,
      (ii) la 2,4-bis[4-(2-éthylhexyloxy)-2-hydroxyphényl]-6-(4-méthoxyphényl)-1,3,5-triazine,
      (iii) le butyl méthoxydibenzoylméthane,
      (iv) l'éthylhexyl triazone, et
      (v) la diéthylhexyl butamido triazone ;

   **(b)** un système solubilisant dudit système photoprotecteur liposoluble (a), comprenant du $C_{12}$-$C_{15}$ alkyl benzoate dans une quantité supérieure ou égale à 80 % en poids, de préférence supérieure ou égale à 90 % en poids, par rapport au poids total du système solubilisant ; et
   **(c)** un ou plusieurs filtre(s) UV organique(s) particulaire(s), en particulier hydrodispersés, choisi(s) parmi :

      (vi) la 5,6,5',6'-tétraphényl-3,3'-(1,4-phénylène)-bis[1,2,4]triazine,
      (vii) le méthylène bis-benzotriazolyl tétraméthylbutylphenol, et
      (viii) la tris-biphényl triazine ;

   **caractérisée en ce que** :

      - le système photoprotecteur liposoluble **(a)** représente entre 3 % et 19 % en poids, de préférence entre 5 % et 15 % en poids, par rapport au poids total de la composition ; et
      - le système solubilisant **(b)** représente entre 15 % et 30 % en poids, par rapport au poids total de la composition ;

   ladite composition ne comprenant pas d'éthylhexyl salicylate, d'octocrylène, d'éthylhexyl méthoxycinnamate, d'isoamyl méthoxylcinnamate, d'homosalate, d'acide para-aminobenzoïque (PABA), d'octyl diméthyl PABA, de 3-méthylbenzylidène camphor, de 4-méthylbenzylidène camphor, de benzophénone-3 et/ou de benzophénone-4.

2. Composition selon la revendication **1, caractérisée en ce que** le système solubilisant **(b)** est constitué de un ou plusieurs émollient(s) lipophile(s) choisi (s) parmi le $C_{12}$-$C_{15}$ alkyl benzoate, les triglycérides caprylique/caprique, le dicaprylyl carbonate, le coco-caprylate, le diisopropyl sébacate, le diéthylhexyl succinate, l'isopropyl palmitate, l'isopropyl myristate, le propylheptyl caprylate, le phénéthyl benzoate, le dibutyl adipate, le diisopropyl adipate, le glycéryl tri-2-éthylhexanoate, le pentaerythrityl tetra-2-éthylhexanoate, le cétyl 2-éthylhexanoate, l'isononyl isononoate, le butylène glycol dicaprylate/dicaprate, l'octyldodécanol, le propylène glycol dicaprylate/dicaprate et le dicaprylyl éther, de préférence parmi le $C_{12}$-$C_{15}$ alkyl benzoate, les triglycérides caprylique/caprique et le dicaprylyl carbonate, en particulier parmi le $C_{12}$-$C_{15}$ alkyl benzoate et les triglycérides caprylique/caprique.

3. Composition selon la revendication 1 ou 2, **caractérisée en ce que** le ou les filtre(s) UV organiques particulaires **(c)** représente(nt) entre 1 % et 8 % en poids, de préférence entre 1 % et 5 % en poids, par rapport au poids total de la composition.

4. Composition selon l'une quelconque des revendications 1 à 3, **caractérisée en ce que** le système photoprotecteur liposoluble **(a)** consiste en l'une des associations de filtres liposolubles suivantes :

   **(a1)** hexyl 2-[4-(diéthylamino)-2-hydroxybenzoyl]benzoate, 2,4-bis[4-(2-éthylhexyloxy)-2-hydroxyphényl]-6-(4-méthoxyphényl)-1,3,5-triazine et éthylhexyl triazone ;
   **(a2)** hexyl 2-[4-(diéthylamino)-2-hydroxybenzoyl]benzoate, 2,4-bis[4-(2-éthylhexyloxy)-2-hydroxyphényl]-6-(4-méthoxyphényl)-1,3,5-triazine et diéthylhexyl butamido triazone ;
   **(a3)** 2,4-bis[4-(2-éthylhexyloxy)-2-hydroxyphényl]-6-(4-méthoxyphényl)-1,3,5-triazine et éthylhexyl triazone ;
   **(a4)** 2,4-bis[4-(2-éthylhexyloxy)-2-hydroxyphényl]-6-(4-méthoxyphényl)-1,3,5-triazine et diéthylhexyl butamido triazone ;
   **(a5)** hexyl 2-[4-(diéthylamino)-2-hydroxybenzoyl]benzoate, butyl méthoxydibenzoylméthane , 2,4-bis[4-(2-éthylhexyloxy)-2-hydroxyphényl]-6-(4-méthoxyphényl)-1,3,5-triazine et éthylhexyl triazone ;
   **(a6)** hexyl 2-[4-(diéthylamino)-2-hydroxybenzoyl]benzoate, butyl méthoxydibenzoylméthane, 2,4-bis[4-(2-éthylhexyloxy)-2-hydroxyphényl]-6-(4-méthoxyphényl)-1,3,5-triazine et diéthylhexyl butamido triazone ;
   **(a7)** butyl méthoxydibenzoylméthane , 2,4-bis[4-(2-éthylhexyloxy)-2-hydroxyphényl]-6-(4-méthoxyphényl)-1,3,5-triazine et éthylhexyl triazone ; et
   **(a8)** butyl méthoxydibenzoylméthane, 2,4-bis[4-(2-éthylhexyloxy)-2-hydroxyphényl]-6-(4-méthoxyphényl)-1,3,5-triazine et diéthylhexyl butamido triazone.

5. Composition selon la revendication 4, **caractérisée en ce que** le système photoprotecteur liposoluble **(a)** consiste en l'association de filtres liposolubles **(a1).**

6. Composition selon l'une quelconque des revendications 1 à 5, **caractérisée en ce que** :

   - le ratio pondéral système photoprotecteur liposoluble **(a)** / système solubilisant **(b)** est supérieur ou égal à 0,5, et/ou
   - le ratio pondéral système photoprotecteur liposoluble **(a)** / $C_{12}$-$C_{15}$ alkyl benzoate est supérieur ou égal à 0,5, et/ou
   - le ratio pondéral filtre(s) UV organique(s) particulaire(s) **(c)** / système photoprotecteur liposoluble **(a)** est inférieur ou égal à 0,35, en particulier inférieur ou égal à 0,33, avantageusement inférieur ou égal à 0,32, notamment inférieur ou égal à 0,30.

7. Composition selon l'une quelconque des revendications 1 à 6, **caractérisée en ce qu'**elle comprend de la 2,4-bis[4-(2-éthylhexyloxy)-2-hydroxyphényl]-6-(4-méthoxyphényl)-1,3,5-triazine en une quantité comprise entre 1 % et 4 % en poids, notamment entre 2 % et 4 %, en particulier entre 2 % et 3 % en poids, par rapport au poids total de la composition.

8. Composition selon l'une quelconque des revendications 1 à 7, **caractérisée en ce qu'**elle comprend de l'hexyl 2-[4-(diéthylamino)-2-hydroxybenzoyl]benzoate en une quantité comprise entre 1 % et 10 % en poids, notamment entre 3 % et 10 % en poids, en particulier entre 4 % et 9 % en poids, typiquement entre 5 % et 8 % en poids, notamment entre 5 % et 7 % en poids, par exemple entre 5 % et 6 % en poids, par rapport au poids total de la composition.

9. Composition selon l'une quelconque des revendications 1 à 8, **caractérisée en ce qu'**elle comprend de l'éthylhexyl triazone en une quantité comprise entre 1 % et 6 %, typiquement entre 2 % et 5 %, notamment entre 3 % et 5 %, en particulier entre 3,5 % et 4,5 % en poids par rapport au poids total de la composition.

10. Composition selon l'une quelconque des revendications 1 à 9, **caractérisée en ce que** le ou les filtre(s) organiques particulaires **(c)** consiste(nt) en un unique filtre organique hydrodispersé qui est le 5,6,5',6'-tétraphényl-3,3'-(1,4-phényléne)-bis[1,2,4]triazine, présentant avantageusement une taille $D_{50}$ de particule comprise entre 100 et 1 000 nm, et représentant avantageusement entre 2 % et 5 % en poids, en particulier entre 2 % et 4 % en poids, typiquement entre 3 % et 4 % en poids, par rapport au poids total de la composition.

11. Composition selon l'une quelconque des revendications 1 à 10, **caractérisée en ce que** le nombre total de filtres UV présents dans la composition est inférieur ou égal à 5, de préférence inférieur ou égal à 4, et **en ce que** l'ensemble des filtres UV présents dans la composition représentent entre 4 % et 25 % en poids, en particulier entre 6 % et 20 % en poids, notamment entre 7 % et 18 % en poids, typiquement entre 8 % et 17 % en poids, par rapport au poids total de la composition.

**12.** Composition selon l'une quelconque des revendications 1 à 11, **caractérisée en ce qu'**elle comprend une quantité d'alcools en $C_1$-$C_4$, tels que l'éthanol et l'isopropanol, inférieure ou égale à 4 % en poids, de préférence inférieure ou égale à 2 % en poids, notamment inférieure ou égale à 1 % en poids, en particulier inférieure ou égale à 0,5 % en poids, par rapport au poids total de la composition.

**13.** Composition selon l'une quelconque des revendications 1 à 12, **caractérisée en ce qu'**elle comprend en outre :

- un ou plusieurs filtre(s) inorganique(s) particulaire(s), tel que du dioxyde de titane ($TiO_2$) et/ou de l'oxyde de zinc (ZnO), et/ou
- un ou plusieurs agent(s) de texture sous forme de poudre(s) choisi (s) parmi les ingrédients de la famille des amidons, tels que les amidons de riz, de maïs et de tapioca ; des talcs et des celluloses, en une quantité représentant entre 1 % et 10 %, de préférence entre 1 % et 5 % en poids par rapport au poids total de la composition.

**14.** Composition selon l'une quelconque des revendications 1 à 13, pour son utilisation pour la protection de la peau contre les rayonnements ultraviolets.

**15.** Utilisation d'une composition selon l'une quelconque des revendications 1 à 13, pour la protection des cheveux contre les rayonnements ultraviolets.

Europäisches
Patentamt

European
Patent Office

Office européen
des brevets

# RAPPORT DE RECHERCHE EUROPEENNE

Numéro de la demande

**EP 22 21 0622**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | DE 10 2004 047286 A1 (BEIERSDORF AG [DE]) 13 avril 2006 (2006-04-13) * alinéas [0019], [0020]; revendications 1,3,4 * * alinéa [0045] * * alinéa [0033]; revendication 10 * ----- | 1-15 | INV. A61K8/37 A61K8/35 A61K8/41 A61K8/49 A61Q17/04 |
| A | DE 10 2008 028664 A1 (BEIERSDORF AG [DE]) 10 décembre 2009 (2009-12-10) * revendications 1,8 * * alinéa [0059] * * alinéa [0020] * ----- | 1-15 | |
| A | WO 2016/012586 A1 (BASF SE [DE]) 28 janvier 2016 (2016-01-28) * exemple B3 * * page 4, ligne 4 - ligne 9 * * page 6, ligne 4 * * page 22, ligne 25 - ligne 27 * ----- | 1-15 | |
| A | FR 2 986 154 A1 (FABRE PIERRE DERMO COSMETIQUE [FR]) 2 août 2013 (2013-08-02) * revendications; exemples * ----- | 1-15 | **DOMAINES TECHNIQUES RECHERCHES (IPC)** A61K A61Q |

Le présent rapport a été établi pour toutes les revendications

| Lieu de la recherche | Date d'achèvement de la recherche | Examinateur |
|---|---|---|
| Munich | 1 mars 2023 | Werner, Stefan |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 22 21 0622

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-03-2023

| Document brevet cité au rapport de recherche | Date de publication | Membre(s) de la famille de brevet(s) | Date de publication |
|---|---|---|---|
| DE 102004047286 A1 | 13-04-2006 | DE 102004047286 A1 | 13-04-2006 |
| | | WO 2006035007 A1 | 06-04-2006 |
| DE 102008028664 A1 | 10-12-2009 | AUCUN | |
| WO 2016012586 A1 | 28-01-2016 | BR 112017001490 A2 | 05-12-2017 |
| | | CN 106659653 A | 10-05-2017 |
| | | CN 115227593 A | 25-10-2022 |
| | | EP 3171945 A1 | 31-05-2017 |
| | | JP 7204715 B2 | 16-01-2023 |
| | | JP 2017521463 A | 03-08-2017 |
| | | JP 2021001211 A | 07-01-2021 |
| | | JP 2022180512 A | 06-12-2022 |
| | | KR 20170034891 A | 29-03-2017 |
| | | US 2018200170 A1 | 19-07-2018 |
| | | WO 2016012586 A1 | 28-01-2016 |
| FR 2986154 A1 | 02-08-2013 | AR 089849 A1 | 24-09-2014 |
| | | AU 2013214293 A1 | 28-08-2014 |
| | | AU 2017239521 A1 | 26-10-2017 |
| | | BR 112014018208 B1 | 03-12-2019 |
| | | CA 2862201 A1 | 08-08-2013 |
| | | CN 104093395 A | 08-10-2014 |
| | | CN 108464951 A | 31-08-2018 |
| | | DK 2809297 T3 | 09-12-2019 |
| | | EP 2809297 A2 | 10-12-2014 |
| | | EP 3626226 A1 | 25-03-2020 |
| | | ES 2759019 T3 | 07-05-2020 |
| | | FR 2986154 A1 | 02-08-2013 |
| | | HK 1199400 A1 | 03-07-2015 |
| | | JP 6563195 B2 | 21-08-2019 |
| | | JP 2015505548 A | 23-02-2015 |
| | | JP 2018135357 A | 30-08-2018 |
| | | KR 20140127272 A | 03-11-2014 |
| | | KR 20190107747 A | 20-09-2019 |
| | | MX 351446 B | 16-10-2017 |
| | | PL 2809297 T3 | 19-07-2021 |
| | | PT 2809297 T | 17-12-2019 |
| | | RU 2014133693 A | 20-03-2016 |
| | | RU 2017136858 A | 21-02-2019 |
| | | TW 201336522 A | 16-09-2013 |
| | | TW 201825075 A | 16-07-2018 |
| | | US 2015050223 A1 | 19-02-2015 |
| | | US 2017348217 A1 | 07-12-2017 |
| | | WO 2013113745 A2 | 08-08-2013 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016012586 A

**Littérature non-brevet citée dans la description**

- **DOUCET et al.** *Toxicology in vitro,* 2006, vol. 20, 499-512